# EUROPEAN PATENT SPECIFICATION

(11) **EP 1 187 925 B1**
(45) Date of publication and mention of the grant of the patent: **30.03.2005**
(21) Application number: 00931043.4
(22) Date of filing: 31.05.2000
(51) Int. Cl.: C12N 15/62, C12N 15/75, C07K 19/00, C12N 9/26

(54) **PECTATE LYASE FUSION FOR EXPRESSION AND SECRETION OF POLYPEPTIDES**
PEKTAT LYASE-FUSION ZUR EXPRESSION UND AUSSCHEIDUNG VON POLYPEPTIDEN
FUSION DE LYASE DE PECTATE PERMETTANT D'EXPRIMER ET DE SECRETER DES POLYPEPTIDES

(30) Priority: 02.06.1999 DK 78099
(43) Date of publication of application: 20.03.2002
(73) Proprietor: Novozymes A/S, 2880 Bagsvaerd (DK)
(72) Inventor: RASMUSSEN, Michael, Dolberg, DK-2625 Vallensbaek (DK); BJOERNVAD, Mads, Eskelund, DK-1955 Frederiksberg (DK); DIERS, Ivan, DK-3500 Vaerloese (DK)
(86) International application number: PCT/DK2000/000296
(87) International publication number: WO 2000/075344

(56) References cited:
- EP-A2- 0 161 937
- EP-A2- 0 683 228
- US-A- 5 846 818
- DATABASE GENBANK [Online] 13 April 1999 PASTOR F.I.J., XP002965642 Retrieved from EMBL Database accession no. AJ237980

### SEQUENCE LISTING

<110> Novo Nordisk A/S
<120> Pectate lyase fusion for expression and secretion of polypeptides.
<130> Pectate lyase fusion proteins
<140>
   <141>
<160> 38
<170> PatentIn Ver. 2.1
<210> 1
   <211> 1026
   <212> DNA
   <213> Bacillus licheniformis ATCC 14580
<220>
   <221> CDS
   <222> (1)..(1026)
<400> 1
   atg aag aaa tta atc agc atc atc ttt atc ttt gta tta ggg gtt gtc     48
   Met Lys Lys Leu Ile Ser Ile Ile Phe Ile Phe Val Leu Gly Val Val
   1               5                   10                  15
   ggg tca ttg aca gcg gcg gtt tcg gca gaa gca gct tct gcc tta aac     96
   Gly Ser Leu Thr Ala Ala Val Ser Ala Glu Ala Ala Ser Ala Leu Asn
               20                  25                  30
   tcg ggc aaa gta aat ccg ctt gcc gac ttc agc tta aaa ggc ttt gcc    144
   Ser Gly Lys Val Asn Pro Leu Ala Asp Phe Ser Leu Lys Gly Phe Ala
           35                  40                  45
   gca cta aac ggc gga aca acg ggc gga gaa ggc ggt cag acg gta acc    192
   Ala Leu Asn Gly Gly Thr Thr Gly Gly Glu Gly Gly Gln Thr Val Thr
       50                  55                  60
   gta aca acg gga gat cag ctg att gcg gca tta aaa aat aag aat gca    240
   Val Thr Thr Gly Asp Gln Leu Ile Ala Ala Leu Lys Asn Lys Asn Ala
   65                  70                  75                  80
   aat acg cct tta aaa att tat gtc aac ggc acc att aca aca tca aat    288
   Asn Thr Pro Leu Lys Ile Tyr Val Asn Gly Thr Ile Thr Thr Ser Asn
                    85                  90                  95
   aca tcc gca tca aag att gac gtc aaa gac gtg tca aac gta tcg att     336
   Thr Ser Ala Ser Lys Ile Asp Val Lys Asp Val Ser Asn Val Ser Ile
               100                 105                 110
   gtc gga tca ggg acc aaa ggg gaa ctc aaa ggg atc ggc atc aaa ata     384
   Val Gly Ser Gly Thr Lys Gly Glu Leu Lys Gly Ile Gly Ile Lys Ile
           115                 120                 125
   tgg cgg gcc aac aac atc atc atc cgc aac ttg aaa att cac gag gtc     432
   Trp Arg Ala Asn Asn Ile Ile Ile Arg Asn Leu Lys Ile His Glu Val
       130                 135                 140
   gcc tca ggc gat aaa gac gcg atc ggc att gaa ggc cct tct aaa aac     480
   Ala Ser Gly Asp Lys Asp Ala Ile Gly Ile Glu Gly Pro Ser Lys Asn
   145                 150                 155                 160
   att tgg gtt gat cat aat gag ctt tac cac agc ctg aac gtt gac aaa     528
   Ile Trp Val Asp His Asn Glu Leu Tyr His Ser Leu Asn Val Asp Lys
                   165                 170                 175
   gat tac tat gac gga tta ttt gac gtc aaa aga gat gcg gaa tat att     576
   Asp Tyr Tyr Asp Gly Leu Phe Asp Val Lys Arg Asp Ala Glu Tyr Ile
               180                 185                 190
   aca ttc tct tgg aac tat gtg cac gat gga tgg aaa tca atg ctg atg     624
   Thr Phe Ser Trp Asn Tyr Val His Asp Gly Trp Lys Ser Met Leu Met
           195                 200                 205
   ggt tca tcg gac agc gat aat tac aac agg acg att aca ttc cat cat     672
   Gly Ser Ser Asp Ser Asp Asn Tyr Asn Arg Thr Ile Thr Phe His His
       210                 215                 220
   aac tgg ttt gag aat ctg aat tcg cgt gtg ccg tca ttc cgt ttc gga     720
   Asn Trp Phe Glu Asn Leu Asn Ser Arg Val Pro Ser Phe Arg Phe Gly
   225                 230                 235                 240
   gaa ggc cat att tac aac aac tat ttc aat aaa atc atc gac agc gga     768
   Glu Gly His Ile Tyr Asn Asn Tyr Phe Asn Lys Ile Ile Asp Ser Gly
                   245                 250                 255
   att aat tcg agg atg ggc gcg cgc atc aga att gag aac aac ctc ttt     816
   Ile Asn Ser Arg Met Gly Ala Arg Ile Arg Ile Glu Asn Asn Leu Phe
               260                 265                 270
   gaa aac gcc aaa gat ccg att gtc tct tgg tac agc agt tca ccg ggc     864
   Glu Asn Ala Lys Asp Pro Ile Val Ser Trp Tyr Ser Ser Ser Pro Gly
           275                 280                 285
   tat tgg cat gta tcc aac aac aaa ttt gta aac tct agg ggc agt atg    912
   Tyr Trp His Val Ser Asn Asn Lys Phe Val Asn Ser Arg Gly Ser Met
       290                 295                 300
   ccg act acc tct act aca acc tat aat ccg cca tac agc tac tca ctc    960
   Pro Thr Thr Ser Thr Thr Thr Tyr Asn Pro Pro Tyr Ser Tyr Ser Leu
   305                 310                 315                 320
   gac aat gtc gac aat gta aaa tca atc gtc aag caa aat gcc gga gtc    1008
   Asp Asn Val Asp Asn Val Lys Ser Ile Val Lys Gln Asn Ala Gly Val
                   325                 330                 335
   ggc aaa atc aat cca taa                                            1026
   Gly Lys Ile Asn Pro
               340
<210> 2
   <211> 341
   <212> PRT
   <213> Bacillus licheniformis ATCC 14580
<400> 2
   Met Lys Lys Leu Ile Ser Ile Ile Phe Ile Phe Val Leu Gly Val Val
   1 5 10 15
   Gly Ser Leu Thr Ala Ala Val Ser Ala Glu Ala Ala Ser Ala Leu Asn
   20 25 30
   Ser Gly Lys Val Asn Pro Leu Ala Asp Phe Ser Leu Lys Gly Phe Ala
   35 40 45
   Ala Leu Asn Gly Gly Thr Thr Gly Gly Glu Gly Gly Gln Thr Val Thr
   50 55 60
   Val Thr Thr Gly Asp Gln Leu Ile Ala Ala Leu Lys Asn Lys Asn Ala
   65 70 75 80
   Asn Thr Pro Leu Lys Ile Tyr Val Asn Gly Thr Ile Thr Thr Ser Asn
   85 90 95
   Thr Ser Ala Ser Lys Ile Asp Val Lys Asp Val Ser Asn Val Ser Ile
   100 105 110
   Val Gly Ser Gly Thr Lys Gly Glu Leu Lys Gly Ile Gly Ile Lys Ile
   115 120 125
   Trp Arg Ala Asn Asn Ile Ile Ile Arg Asn Leu Lys Ile His Glu Val
   130 135 140
   Ala Ser Gly Asp Lys Asp Ala Ile Gly Ile Glu Gly Pro Ser Lys Asn
   145 150 155 160
   Ile Trp Val Asp His Asn Glu Leu Tyr His Ser Leu Asn Val Asp Lys
   165 170 175
   Asp Tyr Tyr Asp Gly Leu Phe Asp Val Lys Arg Asp Ala Glu Tyr Ile
   180 185 190
   Thr Phe Ser Trp Asn Tyr Val His Asp Gly Trp Lys Ser Met Leu Met
   195 200 205
   Gly Ser Ser Asp Ser Asp Asn Tyr Asn Arg Thr Ile Thr Phe His His
   210 215 220
   Asn Trp Phe Glu Asn Leu Asn Ser Arg Val Pro Ser Phe Arg Phe Gly
   225 230 235 240
   Glu Gly His Ile Tyr Asn Asn Tyr Phe Asn Lys Ile Ile Asp Ser Gly
   245 250 255
   Ile Asn Ser Arg Met Gly Ala Arg Ile Arg Ile Glu Asn Asn Leu Phe
   260 265 270
   Glu Asn Ala Lys Asp Pro Ile Val Ser Trp Tyr Ser Ser Ser Pro Gly
   275 280 285
   Tyr Trp His Val Ser Asn Asn Lys Phe Val Asn Ser Arg Gly Ser Met
   290 295 300
   Pro Thr Thr Ser Thr Thr Thr Tyr Asn Pro Pro Tyr Ser Tyr Ser Leu
   305 310 315 320
   Asp Asn Val Asp Asn Val Lys Ser Ile Val Lys Gln Asn Ala Gly Val
   325 330 335
   Gly Lys Ile Asn Pro
   340
<210> 3
   <211> 1530
   <212> DNA
   <213> Bacillus sp.
<220>
   <221> CDS
   <222> (1)..(1530)
<400> 3
   atg atg aag atg aga aaa gca tta agt gta tta gtg att ttc gga tta    48
   Met Met Lys Met Arg Lys Ala Leu Ser Val Leu Val Ile Phe Gly Leu
     1               5                  10                  15
   ttc gta tct ttt ttt agt ttt ggt cat caa gga gca gaa gcg gca tca    96
   Phe Val Ser Phe Phe Ser Phe Gly His Gln Gly Ala Glu Ala Ala Ser
                20                  25                  30
   ttt cag tct aat aaa aat tat cat cta gtg aat gtg aac agt ggc aag   144
   Phe Gln Ser Asn Lys Asn Tyr His Leu Val Asn Val Asn Ser Gly Lys
            35                  40                  45
   tac tta gaa gtg ggg gct gcc tca aca gag aac ggt gca aat gtc caa   192
   Tyr Leu Glu Val Gly Ala Ala Ser Thr Glu Asn Gly Ala Asn Val Gln
           50                  55                  60
   caa tgg gaa aat acg aat tgt cat tgt caa caa tgg cga ttg gtg caa     240
   Gln Trp Glu Asn Thr Asn Cys His Cys Gln Gln Trp Arg Leu Val Gln
    65                  70                  75                  80
   aat cag gat ggt tat tat gag att gta aac cga cat agt ggc aaa gca     288
   Asn Gln Asp Gly Tyr Tyr Glu Ile Val Asn Arg His Ser Gly Lys Ala
                    85                  90                  95
   ttg gat gta ttt gaa cgt tct tca gct gat gga gcg aac att gta caa     336
   Leu Asp Val Phe Glu Arg Ser Ser Ala Asp Gly Ala Asn Ile Val Gln
               100                 105                 110
   tgg gat tcg aat gga cgt agc aat caa caa tgg acg att caa caa gtg     384
   Trp Asp Ser Asn Gly Arg Ser Asn Gln Gln Trp Thr Ile Gln Gln Val
           115                 120                 125
   ggt tcc tct tat aaa ata gtt agc aga cat agt ggg aag gca ctc gaa     432
   Gly Ser Ser Tyr Lys Ile Val Ser Arg His Ser Gly Lys Ala Leu Glu
       130                 135                 140
   gta ttt aac cat tct aat caa aat gga gca aat gtc gta cag tgg caa     480
   Val Phe Asn His Ser Asn Gln Asn Gly Ala Asn Val Val Gln Trp Gln
   145                 150                 155                 160
   gat ttt ggt aat ccg aat caa ctt tgg aat atc gtc gag gtt ggt tca     528
   Asp Phe Gly Asn Pro Asn Gln Leu Trp Asn Ile Val Glu Val Gly Ser
                   165                 170                 175
   gga caa gct cac gat ttc agt aag ccg ttg ggg tat gcc tca atg aat     576
   Gly Gln Ala His Asp Phe Ser Lys Pro Leu Gly Tyr Ala Ser Met Asn
               180                 185                 190
   ggc ggg acc act ggc ggt caa ggt gga cga gtc gaa tac gcg agt act     624
   Gly Gly Thr Thr Gly Gly Gln Gly Gly Arg Val Glu Tyr Ala Ser Thr
           195                 200                 205
   ggc tct caa cta caa aaa tta atc gat gat cga agt cga agc aat aat     672
   Gly Ser Gln Leu Gln Lys Leu Ile Asp Asp Arg Ser Arg Ser Asn Asn
       210                 215                 220
   ccc aat caa cca ctt acc att tat gta act ggg aaa atc acc ctg caa     720
   Pro Asn Gln Pro Leu Thr Ile Tyr Val Thr Gly Lys Ile Thr Leu Gln
   225                 230                 235                 240
   aac tcc tct gat gat aaa att gaa gtg aaa aat cat cgt gga caa gct     768
   Asn Ser Ser Asp Asp Lys Ile Glu Val Lys Asn His Arg Gly Gln Ala
                   245                 250                 255
   cat gaa ata cgt aat ctg tct atc ata ggt caa gga aca aga gga gag     816
   His Glu Ile Arg Asn Leu Ser Ile Ile Gly Gln Gly Thr Arg Gly Glu
               260                 265                 270
   ttt gat ggc att ggt tta cga tta att aat gcg cac aat gtc att gtg     864
   Phe Asp Gly Ile Gly Leu Arg Leu Ile Asn Ala His Asn Val Ile Val
           275                 280                 285
   cgt aat ctc tcc att cac cat gta cga gct ggt tca ggt gaa ggt aca     912
   Arg Asn Leu Ser Ile His His Val Arg Ala Gly Ser Gly Glu Gly Thr
       290                 295                 300
   tca att gaa gtt act caa gga agt aag aat att tgg att gat cat aac     960
   Ser Ile Glu Val Thr Gln Gly Ser Lys Asn Ile Trp Ile Asp His Asn
   305                 310                 315                 320
   gaa ttt tat agt caa ctg gat ggg aat aac aac cct gat ctg tat gat    1008
   Glu Phe Tyr Ser Gln Leu Asp Gly Asn Asn Asn Pro Asp Leu Tyr Asp
                   325                 330                 335
   ggt ctt gtc gat att aaa cgg aat tcg gag tac att acg gtc tct tgg    1056
   Gly Leu Val Asp Ile Lys Arg Asn Ser Glu Tyr Ile Thr Val Ser Trp
               340                 345                 350
   aac aag ttt gag aat cat tgg aaa acg atg ctc gtc ggc cat acc gat    1104
   Asn Lys Phe Glu Asn His Trp Lys Thr Met Leu Val Gly His Thr Asp
           355                 360                 365
   aac gca tca tta gca cct gat aaa gtt acg tac cac cac aac ttt ttc    1152
   Asn Ala Ser Leu Ala Pro Asp Lys Val Thr Tyr His His Asn Phe Phe
       370                 375                 380
   cac aat ctt aat tcc aga gtt ccg tta att cga ttc gca gat gtt cat    1200
   His Asn Leu Asn Ser Arg Val Pro Leu Ile Arg Phe Ala Asp Val His
   385                 390                 395                 400
   atg gtt aac aac tat ttc aaa gat att aaa gat aca gca att aat agt    1248
   Met Val Asn Asn Tyr Phe Lys Asp Ile Lys Asp Thr Ala Ile Asn Ser
                   405                 410                 415
   cgt atg gga gca aga gta ttt gta gaa aat aac tat ttt gag aat gta    1296
   Arg Met Gly Ala Arg Val Phe Val Glu Asn Asn Tyr Phe Glu Asn Val
               420                 425                 430
   gga tca ggt caa caa gat ccg acc aca cga caa att aaa act gct gtt    1344
   Gly Ser Gly Gln Gln Asp Pro Thr Thr Arg Gln Ile Lys Thr Ala Val
           435                 440                 445
   ggg tgg ttt tat ggt agt tct agc act gga tat tgg aat tta aga gga   1392
   Gly Trp Phe Tyr Gly Ser Ser Ser Thr Gly Tyr Trp Asn Leu Arg Gly
       450                 455                 460
   aat caa ttt att aac aca cca tca agc cac ttg tct tcc aca acg aat   1440
   Asn Gln Phe Ile Asn Thr Pro Ser Ser His Leu Ser Ser Thr Thr Asn
   465                 470                 475                 480
   ttc aca cca cct tat cag ttc aac gcc caa tcc gct caa gat gca aag   1488
   Phe Thr Pro Pro Tyr Gln Phe Asn Ala Gln Ser Ala Gln Asp Ala Lys
                   485                 490                 495
   caa gcc gtt gaa cag ttt tcg ggt gta ggg gtt gta cag tag           1530
   Gln Ala Val Glu Gln Phe Ser Gly Val Gly Val Val Gln
               500                 505                 510
<210> 4
   <211> 509
   <212> PRT
   <213> Bacillus sp.
<400> 4
   Met Met Lys Met Arg Lys Ala Leu Ser Val Leu Val Ile Phe Gly Leu
    1               5                  10                  15
   Phe Val Ser Phe Phe Ser Phe Gly His Gln Gly Ala Glu Ala Ala Ser
               20                  25                  30
   Phe Gln Ser Asn Lys Asn Tyr His Leu Val Asn Val Asn Ser Gly Lys
           35                  40                  45
   Tyr Leu Glu Val Gly Ala Ala Ser Thr Glu Asn Gly Ala Asn Val Gln
       50                  55                  60
   Gln Trp Glu Asn Thr Asn Cys His Cys Gln Gln Trp Arg Leu Val Gln
   65                  70                  75                  80
   Asn Gln Asp Gly Tyr Tyr Glu Ile Val Asn Arg His Ser Gly Lys Ala
                   85                  90                  95
   Leu Asp Val Phe Glu Arg Ser Ser Ala Asp Gly Ala Asn Ile Val Gln
               100                 105                 110
   Trp Asp Ser Asn Gly Arg Ser Asn Gln Gln Trp Thr Ile Gln Gln Val
           115                 120                 125
   Gly Ser Ser Tyr Lys Ile Val Ser Arg His Ser Gly Lys Ala Leu Glu
       130                 135                 140
   Val Phe Asn His Ser Asn Gln Asn Gly Ala Asn Val Val Gln Trp Gln
   145                 150                 155                 160
   Asp Phe Gly Asn Pro Asn Gln Leu Trp Asn Ile Val Glu Val Gly Ser
                   165                 170                 175
   Gly Gln Ala His Asp Phe Ser Lys Pro Leu Gly Tyr Ala Ser Met Asn
                       180                     185                     190
   Gly Gly Thr Thr Gly Gly Gln Gly Gly Arg Val Glu Tyr Ala Ser Thr
           195                     200                     205
   Gly Ser Gln Leu Gln Lys Leu Ile Asp Asp Arg Ser Arg Ser Asn Asn
       210                     215                     220
   Pro Asn Gln Pro Leu Thr Ile Tyr Val Thr Gly Lys Ile Thr Leu Gln
   225                     230                     235                 240
   Asn Ser Ser Asp Asp Lys Ile Glu Val Lys Asn His Arg Gly Gln Ala
                   245                     250                     255
   His Glu Ile Arg Asn Leu Ser Ile Ile Gly Gln Gly Thr Arg Gly Glu
               260                     265                     270
   Phe Asp Gly Ile Gly Leu Arg Leu Ile Asn Ala His Asn Val Ile Val
           275                     280                     285
   Arg Asn Leu Ser Ile His His Val Arg Ala Gly Ser Gly Glu Gly Thr
       290                     295                     300
   Ser Ile Glu Val Thr Gln Gly Ser Lys Asn Ile Trp Ile Asp His Asn
   305                     310                     315                 320
   Glu Phe Tyr Ser Gln Leu Asp Gly Asn Asn Asn Pro Asp Leu Tyr Asp
                   325                     330                     335
   Gly Leu Val Asp Ile Lys Arg Asn Ser Glu Tyr Ile Thr Val Ser Trp
               340                     345                     350
   Asn Lys Phe Glu Asn His Trp Lys Thr Met Leu Val Gly His Thr Asp
           355                     360                     365
   Asn Ala Ser Leu Ala Pro Asp Lys Val Thr Tyr His His Asn Phe Phe
       370                     375                     380
   His Asn Leu Asn Ser Arg Val Pro Leu Ile Arg Phe Ala Asp Val His
   385                     390                     395                 400
   Met Val Asn Asn Tyr Phe Lys Asp Ile Lys Asp Thr Ala Ile Asn Ser
                   405                     410                     415
   Arg Met Gly Ala Arg Val Phe Val Glu Asn Asn Tyr Phe Glu Asn Val
               420                     425                     430
   Gly Ser Gly Gln Gln Asp Pro Thr Thr Arg Gln Ile Lys Thr Ala Val
           435                     440                     445
   Gly Trp Phe Tyr Gly Ser Ser Ser Thr Gly Tyr Trp Asn Leu Arg Gly
       450                     455                     460
   Asn Gln Phe Ile Asn Thr Pro Ser Ser His Leu Ser Ser Thr Thr Asn
   465                     470                     475                 480
   Phe Thr Pro Pro Tyr Gln Phe Asn Ala Gln Ser Ala Gln Asp Ala Lys
                   485                     490                     495
   Gln Ala Val Glu Gln Phe Ser Gly Val Gly Val Val Gln
               500                     505
<210> 5
   <211> 1008
   <212> DNA
   <213> Bacillus sp.
   <220>
   **<221>** CDS
   <222> (1)..(1008)
<400> 5
   atg aga aaa ctc tta tcg atg atg act gcg ctt gta ctc atg ttt gga    48
   Met Arg Lys Leu Leu Ser Met Met Thr Ala Leu Val Leu Met Phe Gly
   1               5                   10                  15
   atc atg gtt gta cct tct ata gcc aaa ggt gaa agc gat tcc act atg    96
   Ile Met Val Val Pro Ser Ile Ala Lys Gly Glu Ser Asp Ser Thr Met
               20                  25                  30
   aat gct gat ttt tcc atg caa ggt ttt gcg aca ctt aat ggc gga acc    144
   Asn Ala Asp Phe Ser Met Gln Gly Phe Ala Thr Leu Asn Gly Gly Thr
           35                  40                  45
   aca gga gga gcc ggc ggg caa acc gta acc gtt tct acc gga gac gaa    192
   Thr Gly Gly Ala Gly Gly Gln Thr Val Thr Val Ser Thr Gly Asp Glu
       50                  55                  60
   ctg ctg gcg gcc ttg aag aac aaa aac agc aat aca ccc ctg acg att    240
   Leu Leu Ala Ala Leu Lys Asn Lys Asn Ser Asn Thr Pro Leu Thr Ile
   65                  70                  75                  80
   tat gta aac ggt acc ata acg cca tca aat acg tcc gca agc aaa att    288
   Tyr Val Asn Gly Thr Ile Thr Pro Ser Asn Thr Ser Ala Ser Lys Ile
                   85                  90                  95
   gat att aaa gac gta aac gat gtt tcg atc tta ggt gtt ggc act caa    336
   Asp Ile Lys Asp Val Asn Asp Val Ser Ile Leu Gly Val Gly Thr Gln
               100                 105                 110
   ggc gaa ttt aac ggc att ggc att aaa gta tgg cga gcc aat aac att    384
   Gly Glu Phe Asn Gly Ile Gly Ile Lys Val Trp Arg Ala Asn Asn Ile
           115                 120                 125
   att ctc cgc aac ttg aaa ata cat cac gtc aat aca ggc gac aaa gat    432
   Ile Leu Arg Asn Leu Lys Ile His His Val Asn Thr Gly Asp Lys Asp
       130                 135                 140
   gcc att agc att gaa gga cca tcc aaa aac ata tgg gtt gac cac aat    480
   Ala Ile Ser Ile Glu Gly Pro Ser Lys Asn Ile Trp Val Asp His Asn
   145                 150                 155                 160
   gag ctc tac aat agt ctt gat gtc cat aag gat tac tac gat ggt ctt    528
   Glu Leu Tyr Asn Ser Leu Asp Val His Lys Asp Tyr Tyr Asp Gly Leu
   165 170 175
   ttt gat gtc aaa cgg gac gcg gat tac att aca ttc tcg tgg aat tat 576
   Phe Asp Val Lys Arg Asp Ala Asp Tyr Ile Thr Phe Ser Trp Asn Tyr
   180 185 190
   gtt cat gat agc tgg aag agc atg ctg atg gga tct tct gat tcc gat 624
   Val His Asp Ser Trp Lys Ser Met Leu Met Gly Ser Ser Asp Ser Asp
   195 200 205
   tcg tac aac cga aaa atc aca ttc cac aat aac tac ttt gaa aac ctc 672
   Ser Tyr Asn Arg Lys Ile Thr Phe His Asn Asn Tyr Phe Glu Asn Leu
   210 215 220
   aat tca cgt gta cct tcc ata cgc ttt ggc gaa gcc cac atc ttc agc 720
   Asn Ser Arg Val Pro Ser Ile Arg Phe Gly Glu Ala His Ile Phe Ser
   225 230 235 240
   aac tac tac aat ggc att aat gaa acc ggc atc aac tcc cgc atg ggg 768
   Asn Tyr Tyr Asn Gly Ile Asn Glu Thr Gly Ile Asn Ser Arg Met Gly
   245 250 255
   gca aaa gtg cgc atc gag gaa aat cta ttt gaa cgc gca aac aac ccg 816
   Ala Lys Val Arg Ile Glu Glu Asn Leu Phe Glu Arg Ala Asn Asn Pro
   260 265 270
   atc gtc agt cgc gac agt cgc caa gtc ggg tat tgg cac ttg ata aac 864
   Ile Val Ser Arg Asp Ser Arg Gln Val Gly Tyr Trp His Leu Ile Asn
   275 280 285
   aat cac ttt act caa tca acg ggc gaa att cca acg act tca aca atc 912
   Asn His Phe Thr Gln Ser Thr Gly Glu Ile Pro Thr Thr Ser Thr Ile
   290 295 300
   aca tat aac cca cct tat tcc tat caa gct act ccg gtt ggc caa gta 960
   Thr Tyr Asn Pro Pro Tyr Ser Tyr Gln Ala Thr Pro Val Gly Gln Val
   305 310 315 320
   aaa gat gtg gtt cgt gcg aat gct ggt gtt ggc aaa gta aca cct taa 1008
   Lys Asp Val Val Arg Ala Asn Ala Gly Val Gly Lys Val Thr Pro
   325 330 335
<210> 6
   <211> 335
   <212> PRT
   <213> Bacillus sp.
<400> 6
   Met Arg Lys Leu Leu Ser Met Met Thr Ala Leu Val Leu Met Phe Gly
   1 5 10 15
   Ile Met Val Val Pro Ser Ile Ala Lys Gly Glu Ser Asp Ser Thr Met
   20 25 30
   Asn Ala Asp Phe Ser Met Gln Gly Phe Ala Thr Leu Asn Gly Gly Thr
   35 40 45
   Thr Gly Gly Ala Gly Gly Gln Thr Val Thr Val Ser Thr Gly Asp Glu
   50 55 60
   Leu Leu Ala Ala Leu Lys Asn Lys Asn Ser Asn Thr Pro Leu Thr Ile
   65 70 75 80
   Tyr Val Asn Gly Thr Ile Thr Pro Ser Asn Thr Ser Ala Ser Lys Ile
   85 90 95
   Asp Ile Lys Asp Val Asn Asp Val Ser Ile Leu Gly Val Gly Thr Gln
   100 105 110
   Gly Glu Phe Asn Gly Ile Gly Ile Lys Val Trp Arg Ala Asn Asn Ile
   115 120 125
   Ile Leu Arg Asn Leu Lys Ile His His Val Asn Thr Gly Asp Lys Asp
   130 135 140
   Ala Ile Ser Ile Glu Gly Pro Ser Lys Asn Ile Trp Val Asp His Asn
   145 150 155 160
   Glu Leu Tyr Asn Ser Leu Asp Val His Lys Asp Tyr Tyr Asp Gly Leu
   165 170 175
   Phe Asp Val Lys Arg Asp Ala Asp Tyr Ile Thr Phe Ser Trp Asn Tyr
   180 185 190
   Val His Asp Ser Trp Lys Ser Met Leu Met Gly Ser Ser Asp Ser Asp
   195 200 205
   Ser Tyr Asn Arg Lys Ile Thr Phe His Asn Asn Tyr Phe Glu Asn Leu
   210 215 220
   Asn Ser Arg Val Pro Ser Ile Arg Phe Gly Glu Ala His Ile Phe Ser
   225 230 235 240
   Asn Tyr Tyr Asn Gly Ile Asn Glu Thr Gly Ile Asn Ser Arg Met Gly
   245 250 255
   Ala Lys Val Arg Ile Glu Glu Asn Leu Phe Glu Arg Ala Asn Asn Pro
   260 265 270
   Ile Val Ser Arg Asp Ser Arg Gln Val Gly Tyr Trp His Leu Ile Asn
   275 280 285
   Asn His Phe Thr Gln Ser Thr Gly Glu Ile Pro Thr Thr Ser Thr Ile
   290 295 300
   Thr Tyr Asn Pro Pro Tyr Ser Tyr Gln Ala Thr Pro Val Gly Gln Val
   305 310 315 320
   Lys Asp Val Val Arg Ala Asn Ala Gly Val Gly Lys Val Thr Pro
   325 330 335
<210> 7
   <211> 1077
   <212> DNA
   <213> Bacillus sp.
<220>
   <221> CDS
   <222> (1)..(1077)
<400> 7
   atg act aaa gtc ttt aaa ttg tta ctg gca tta gct ctc gtt tta cca     48
   Met Thr Lys Val Phe Lys Leu Leu Leu Ala Leu Ala Leu Val Leu Pro
   1               5                   10                  15
   gtt atc tca ttt agt tct cct gcc tcg caa gct gct tca aat cag cca     96
   Val Ile Ser Phe Ser Ser Pro Ala Ser Gln Ala Ala Ser Asn Gln Pro
               20                  25                  30
   act tct aac gga cca caa ggc tat gcg tca atg aat gga ggg aca acc    144
   Thr Ser Asn Gly Pro Gln Gly Tyr Ala Ser Met Asn Gly Gly Thr Thr
           35                  40                  45
   ggt ggt gca ggc ggc cgt gtc gaa tat gca agc acc gga gcg caa att    192
   Gly Gly Ala Gly Gly Arg Val Glu Tyr Ala Ser Thr Gly Ala Gln Ile
       50                  55                  60
   cag caa ttg ata gat aat cgc agc cga agt aat aac cct gat gaa cca    240
   Gln Gln Leu Ile Asp Asn Arg Ser Arg Ser Asn Asn Pro Asp Glu Pro
   65                  70                  75                  80
   tta acg att tat gta aac gga acg att aca caa gga aat tcc cca cag    288
   Leu Thr Ile Tyr Val Asn Gly Thr Ile Thr Gln Gly Asn Ser Pro Gln
                   85                  90                  95
   tcc ctt ata gat gtt aaa aat cac cgt gga aaa gct cat gaa att aaa    336
   Ser Leu Ile Asp Val Lys Asn His Arg Gly Lys Ala His Glu Ile Lys
               100                 105                 110
   aac atc tct att atc ggt gta gga aca aat gga gag ttt gat ggc att    384
   Asn Ile Ser Ile Ile Gly Val Gly Thr Asn Gly Glu Phe Asp Gly Ile
           115                 120                 125
   ggg ata aga cta tca aac gcc cat aat atc att atc caa aat gta tca    432
   Gly Ile Arg Leu Ser Asn Ala His Asn Ile Ile Ile Gln Asn Val Ser
       130                 135                 140
   att cat cat gtg cga gag gga gaa ggc acg gct att gaa gtg aca gat    480
   Ile His His Val Arg Glu Gly Glu Gly Thr Ala Ile Glu Val Thr Asp
   145                 150                 155                 160
   gag agt aaa aac gtg tgg atc gat cac aac gag ttt tat agt gaa ttt     528
   Glu Ser Lys Asn Val Trp Ile Asp His Asn Glu Phe Tyr Ser Glu Phe
                   165                 170                 175
   cca ggt aat gga gac tca gat tat tac gat ggt ctc gta gac ata aaa     576
   Pro Gly Asn Gly Asp Ser Asp Tyr Tyr Asp Gly Leu Val Asp Ile Lys
               180                 185                 190
   aga aac gct gaa tat att acg gtt tca tgg aat aag ttt gag aat cat     624
   Arg Asn Ala Glu Tyr Ile Thr Val Ser Trp Asn Lys Phe Glu Asn His
           195                 200                 205
   tgg aaa acg atg ctc gtc ggt cat act gat aat gcc tca tta gcg cca     672
   Trp Lys Thr Met Leu Val Gly His Thr Asp Asn Ala Ser Leu Ala Pro
       210                 215                 220
   gat aaa att acg tac cat cac aat tat ttt aat aat ctt aat tca cgt     720
   Asp Lys Ile Thr Tyr His His Asn Tyr Phe Asn Asn Leu Asn Ser Arg
   225                 230                 235                 240
   gtc ccg ctt att cga tac gct gat gtc cat atg ttc aat aac tat ttt     768
   Val Pro Leu Ile Arg Tyr Ala Asp Val His Met Phe Asn Asn Tyr Phe
                   245                 250                 255
   aaa gac att aac gat aca gcg att aac agt cgt gta ggg gcc cgt gtc     816
   Lys Asp Ile Asn Asp Thr Ala Ile Asn Ser Arg Val Gly Ala Arg Val
               260                 265                 270
   ttt gta gaa aac aac tat ttt gac aac gta gga tca gga caa gct gac     864
   Phe Val Glu Asn Asn Tyr Phe Asp Asn Val Gly Ser Gly Gln Ala Asp
           275                 280                 285
   cca acg act ggt ttt att aaa ggg cct gtt ggt tgg ttc tat gga agt     912
   Pro Thr Thr Gly Phe Ile Lys Gly Pro Val Gly Trp Phe Tyr Gly Ser
       290                 295                 300
   ccg agt act gga tat tgg aat tta cgt gga aat gta ttt gtt aat aca     960
   Pro Ser Thr Gly Tyr Trp Asn Leu Arg Gly Asn Val Phe Val Asn Thr
   305                 310                 315                 320
   ccg aat agt cat tta agc tct aca aca aac ttt aca cca cca tat agt    1008
   Pro Asn Ser His Leu Ser Ser Thr Thr Asn Phe Thr Pro Pro Tyr Ser
                   325                 330                 335
   tac aaa gtc caa tca gct acc caa gct aag tcg tcg gtt gaa caa cat    1056
   Tyr Lys Val Gln Ser Ala Thr Gln Ala Lys Ser Ser Val Glu Gln His
               340                 345                 350
   tcg gga gta ggt gtt atc aac                                          1077
   Ser Gly Val Gly Val Ile Asn
           355
<210> 8
   <211> 359
   <212> PRT
   <213> Bacillus sp.
<400> 8
   Met Thr Lys Val Phe Lys Leu Leu Leu Ala Leu Ala Leu Val Leu Pro
   1               5                   10                  15
   Val Ile Ser Phe Ser Ser Pro Ala Ser Gln Ala Ala Ser Asn Gln Pro
               20                  25                  30
   Thr Ser Asn Gly Pro Gln Gly Tyr Ala Ser Met Asn Gly Gly Thr Thr
           35                  40                  45
   Gly Gly Ala Gly Gly Arg Val Glu Tyr Ala Ser Thr Gly Ala Gln Ile
       50                  55                  60
   Gln Gln Leu Ile Asp Asn Arg Ser Arg Ser Asn Asn Pro Asp Glu Pro
   65                  70                  75                  80
   Leu Thr Ile Tyr Val Asn Gly Thr Ile Thr Gln Gly Asn Ser Pro Gln
                   85                  90                  95
   Ser Leu Ile Asp Val Lys Asn His Arg Gly Lys Ala His Glu Ile Lys
               100                 105                 110
   Asn Ile Ser Ile Ile Gly Val Gly Thr Asn Gly Glu Phe Asp Gly Ile
           115                 120                 125
   Gly Ile Arg Leu Ser Asn Ala His Asn Ile Ile Ile Gln Asn Val Ser
       130                 135                 140
   Ile His His Val Arg Glu Gly Glu Gly Thr Ala Ile Glu Val Thr Asp
   145                 150                 155                 160
   Glu Ser Lys Asn Val Trp Ile Asp His Asn Glu Phe Tyr Ser Glu Phe
                   165                 170                 175
   Pro Gly Asn Gly Asp Ser Asp Tyr Tyr Asp Gly Leu Val Asp Ile Lys
               180                 185                 190
   Arg Asn Ala Glu Tyr Ile Thr Val Ser Trp Asn Lys Phe Glu Asn His
   195 200 205
   Trp Lys Thr Met Leu Val Gly His Thr Asp Asn Ala Ser Leu Ala Pro
   210 215 220
   Asp Lys Ile Thr Tyr His His Asn Tyr Phe Asn Asn Leu Asn Ser Arg
   225 230 235 240
   Val Pro Leu Ile Arg Tyr Ala Asp Val His Met Phe Asn Asn Tyr Phe
   245 250 255
   Lys Asp Ile Asn Asp Thr Ala Ile Asn Ser Arg Val Gly Ala Arg Val
   260 265 270
   Phe Val Glu Asn Asn Tyr Phe Asp Asn Val Gly Ser Gly Gln Ala Asp
   275 280 285
   Pro Thr Thr Gly Phe Ile Lys Gly Pro Val Gly Trp Phe Tyr Gly Ser
   290 295 300
   Pro Ser Thr Gly Tyr Trp Asn Leu Arg Gly Asn Val Phe Val Asn Thr
   305 310 315 320
   Pro Asn Ser His Leu Ser Ser Thr Thr Asn Phe Thr Pro Pro Tyr Ser
   325 330 335
   Tyr Lys Val Gln Ser Ala Thr Gln Ala Lys Ser Ser Val Glu Gln His
   340 345 350
   Ser Gly Val Gly Val Ile Asn
   355
<210> 9
   <211> 1047
   <212> DNA
   <213> Bacillus sp.
<220>
   <221> CDS
   <222> (1)..(1047)
<400> 9
   atg gac aaa ctt tat att gaa aaa gga agt gag agt atg atg aga tca 48
   Met Asp Lys Leu Tyr Ile Glu Lys Gly Ser Glu Ser Met Met Arg Ser
   1 5 10 15
   agc atc gtc aaa cta gtt gct ttc agt att gtg ttt atg tta tgg ctc 96
   Ser Ile Val Lys Leu Val Ala Phe Ser Ile Val Phe Met Leu Trp Leu
   20 25 30
   ggt gta tcc ttt caa acg gca gaa gcg aat acg cca aat ttc aac tta 144
   Gly Val Ser Phe Gln Thr Ala Glu Ala Asn Thr Pro Asn Phe Asn Leu
   35 40 45
   caa ggc ttt gcc acg tta aat ggg gga aca act ggt ggc gct ggt gga 192
   Gln Gly Phe Ala Thr Leu Asn Gly Gly Thr Thr Gly Gly Ala Gly Gly
   50 55 60
   gat gta gtg acg gtt cgt aca ggg aat gag tta ata aac gct ttg aag 240
   Asp Val Val Thr Val Arg Thr Gly Asn Glu Leu Ile Asn Ala Leu Lys
   65 70 75 80
   tcc aaa aac cct aat cgg ccg tta aca att tat gtt aac ggt acg ata 288
   Ser Lys Asn Pro Asn Arg Pro Leu Thr Ile Tyr Val Asn Gly Thr Ile
   85 90 95
   acg cct aat aat acg tct gat agt aag atc gac att aag gat gtt tcc 336
   Thr Pro Asn Asn Thr Ser Asp Ser Lys Ile Asp Ile Lys Asp Val Ser
   100 105 110
   aat gta tcg att tta ggg gtt ggc aca aat ggc cga tta aac ggg atc 384
   Asn Val Ser Ile Leu Gly Val Gly Thr Asn Gly Arg Leu Asn Gly Ile
   115 120 125
   ggt att aaa gta tgg cga gcg aat aat atc att att cga aac ttg aca 432
   Gly Ile Lys Val Trp Arg Ala Asn Asn Ile Ile Ile Arg Asn Leu Thr
   130 135 140
   atc cat gaa gtc cat aca ggt gat aaa gat gcg att agc atg att agc 480
   Ile His Glu Val His Thr Gly Asp Lys Asp Ala Ile Ser Met Ile Ser
   145 150 155 160
   att gaa gga cca tct cga aac att tgg gtt gac cat aac gag ctt tat 528
   Ile Glu Gly Pro Ser Arg Asn Ile Trp Val Asp His Asn Glu Leu Tyr
   165 170 175
   gcc agc ttg aat gtt cat aaa gat cac tat gac ggc ttg ttt gac gta 576
   Ala Ser Leu Asn Val His Lys Asp His Tyr Asp Gly Leu Phe Asp Val
   180 185 190
   aag cgc gat gct tac aat att acc ttc tct tgg aat tat gtc cat gat 624
   Lys Arg Asp Ala Tyr Asn Ile Thr Phe Ser Trp Asn Tyr Val His Asp
   195 200 205
   ggc tgg aaa gcg atg ctc atg ggg aat tcc gat agt gat aat tat gac    672
   Gly Trp Lys Ala Met Leu Met Gly Asn Ser Asp Ser Asp Asn Tyr Asp
       210                 215                 220
   cga aac ata aca ttc cac cat aac tac ttc aaa aac tta aac tct cgt    720
   Arg Asn Ile Thr Phe His His Asn Tyr Phe Lys Asn Leu Asn Ser Arg
   225                 230                 235                 240
   gta cct gcg tac cgt ttt gga aag gcg cac ttg ttt agc aat tac ttt    768
   Val Pro Ala Tyr Arg Phe Gly Lys Ala His Leu Phe Ser Asn Tyr Phe
                   245                 250                 255
   gag aac att tta gaa aca ggc atc aat tca cgg atg gga gcg gaa atg    816
   Glu Asn Ile Leu Glu Thr Gly Ile Asn Ser Arg Met Gly Ala Glu Met
               260                 265                 270
   ctc gtt gaa cat aac gtt ttt gag aat gcc acc aac ccg cta gga ttc    864
   Leu Val Glu His Asn Val Phe Glu Asn Ala Thr Asn Pro Leu Gly Phe
           275                 280                 285
   tgg cat agc agt cga aca ggt tat tgg aat gta gcc aat aac cgc tat    912
   Trp His Ser Ser Arg Thr Gly Tyr Trp Asn Val Ala Asn Asn Arg Tyr
       290                 295                 300
   atc aat agc acg ggc agc atg ccg acc act tcc acg acc aat tat cga    960
   Ile Asn Ser Thr Gly Ser Met Pro Thr Thr Ser Thr Thr Asn Tyr Arg
   305                 310                 315                 320
   cct cct tac ccc tat acg gtc aca cct gtt ggt gat gtg aaa tca gtt    1008
   Pro Pro Tyr Pro Tyr Thr Val Thr Pro Val Gly Asp Val Lys Ser Val
                   325                 330                 335
   gtc aca cgt tat gcg gga gtt ggt gtc atc caa ccg taa                1047
   Val Thr Arg Tyr Ala Gly Val Gly Val Ile Gln Pro
               340                 345
<210> 10
   <211> 348
   <212> PRT
   <213> Bacillus sp.
<400> 10
   Met Asp Lys Leu Tyr Ile Glu Lys Gly Ser Glu Ser Met Met Arg Ser
   1 5 10 15
   Ser Ile Val Lys Leu Val Ala Phe Ser Ile Val Phe Met Leu Trp Leu
   20 25 30
   Gly Val Ser Phe Gln Thr Ala Glu Ala Asn Thr Pro Asn Phe Asn Leu
   35 40 45
   Gln Gly Phe Ala Thr Leu Asn Gly Gly Thr Thr Gly Gly Ala Gly Gly
   50 55 60
   Asp Val Val Thr Val Arg Thr Gly Asn Glu Leu Ile Asn Ala Leu Lys
   65 70 75 80
   Ser Lys Asn Pro Asn Arg Pro Leu Thr Ile Tyr Val Asn Gly Thr Ile
   85 90 95
   Thr Pro Asn Asn Thr Ser Asp Ser Lys Ile Asp Ile Lys Asp Val Ser
   100 105 110
   Asn Val Ser Ile Leu Gly Val Gly Thr Asn Gly Arg Leu Asn Gly Ile
   115 120 125
   Gly Ile Lys Val Trp Arg Ala Asn Asn Ile Ile Ile Arg Asn Leu Thr
   130 135 140
   Ile His Glu Val His Thr Gly Asp Lys Asp Ala Ile Ser Met Ile Ser
   145 150 155 160
   Ile Glu Gly Pro Ser Arg Asn Ile Trp Val Asp His Asn Glu Leu Tyr
   165 170 175
   Ala Ser Leu Asn Val His Lys Asp His Tyr Asp Gly Leu Phe Asp Val
   180 185 190
   Lys Arg Asp Ala Tyr Asn Ile Thr Phe Ser Trp Asn Tyr Val His Asp
   195 200 205
   Gly Trp Lys Ala Met Leu Met Gly Asn Ser Asp Ser Asp Asn Tyr Asp
   210 215 220
   Arg Asn Ile Thr Phe His His Asn Tyr Phe Lys Asn Leu Asn Ser Arg
   225 230 235 240
   Val Pro Ala Tyr Arg Phe Gly Lys Ala His Leu Phe Ser Asn Tyr Phe
   245 250 255
   Glu Asn Ile Leu Glu Thr Gly Ile Asn Ser Arg Met Gly Ala Glu Met
   260 265 270
   Leu Val Glu His Asn Val Phe Glu Asn Ala Thr Asn Pro Leu Gly Phe
   275 280 285
   Trp His Ser Ser Arg Thr Gly Tyr Trp Asn Val Ala Asn Asn Arg Tyr
   290 295 300
   Ile Asn Ser Thr Gly Ser Met Pro Thr Thr Ser Thr Thr Asn Tyr Arg
   305 310 315 320
   Pro Pro Tyr Pro Tyr Thr Val Thr Pro Val Gly Asp Val Lys Ser Val
   325 330 335
   Val Thr Arg Tyr Ala Gly Val Gly Val Ile Gln Pro
   340 345
<210> 11
   <211> 2502
   <212> DNA
   <213> Artificial Sequence
<220>
   <221> CDS
   <222> (1)..(2502)
<220>
   <223> Description of Artificial Sequence:Details available in the text.
<400> 11
   atg aaa caa caa aaa cgg ctt tac gcc cga ttg ctg acg ctg tta ttt    48
   Met Lys Gln Gln Lys Arg Leu Tyr Ala Arg Leu Leu Thr Leu Leu Phe
     1               5                  10                  15
   gcg ctc atc ttc ttg ctg cct cat tct gca gcc gcg gca gct tct gcc    96
   Ala Leu Ile Phe Leu Leu Pro His Ser Ala Ala Ala Ala Ala Ser Ala
                20                  25                  30
   tta aac tcg ggc aaa gta aat ccg ctt gcc gac ttc agc tta aaa ggc   144
   Leu Asn Ser Gly Lys Val Asn Pro Leu Ala Asp Phe Ser Leu Lys Gly
            35                  40                  45
   ttt gcc gca cta aac ggc gga aca acg ggc gga gaa ggc ggt cag acg   192
   Phe Ala Ala Leu Asn Gly Gly Thr Thr Gly Gly Glu Gly Gly Gln Thr
        50                  55                  60
   gta acc gta aca acg gga gat cag ctg att gcg gca tta aaa aat aag   240
   Val Thr Val Thr Thr Gly Asp Gln Leu Ile Ala Ala Leu Lys Asn Lys
    65                  70                  75                  80
   aat gca aat acg cct tta aaa att tat gtc aac ggc acc att aca aca   288
   Asn Ala Asn Thr Pro Leu Lys Ile Tyr Val Asn Gly Thr Ile Thr Thr
                    85                  90                  95
   tca aat aca tcc gca tca aag att gac gtc aaa gac gtg tca aac gta   336
   Ser Asn Thr Ser Ala Ser Lys Ile Asp Val Lys Asp Val Ser Asn Val
               100                 105                 110
   tcg att gtc gga tca ggg acc aaa ggg gaa ctc aaa ggg atc ggc atc   384
   Ser Ile Val Gly Ser Gly Thr Lys Gly Glu Leu Lys Gly Ile Gly Ile
           115                 120                 125
   aaa ata tgg cgg gcc aac aac atc atc atc cgc aac ttg aaa att cac   432
   Lys Ile Trp Arg Ala Asn Asn Ile Ile Ile Arg Asn Leu Lys Ile His
       130                 135                 140
   gag gtc gcc tca ggc gat aaa gac gcg atc ggc att gaa ggc cct tct   480
   Glu Val Ala Ser Gly Asp Lys Asp Ala Ile Gly Ile Glu Gly Pro Ser
   145                 150                 155                 160
   aaa aac att tgg gtt gat cat aat gag ctt tac cac agc ctg aac gtt    528
   Lys Asn Ile Trp Val Asp His Asn Glu Leu Tyr His Ser Leu Asn Val
                   165                 170                 175
   gac aaa gat tac tat gac gga tta ttt gac gtc aaa aga gat gcg gaa    576
   Asp Lys Asp Tyr Tyr Asp Gly Leu Phe Asp Val Lys Arg Asp Ala Glu
               180                 185                 190
   tat att aca ttc tct tgg aac tat gtg cac gat gga tgg aaa tca atg    624
   Tyr Ile Thr Phe Ser Trp Asn Tyr Val His Asp Gly Trp Lys Ser Met
           195                 200                 205
   ctg atg ggt tca tcg gac agc gat aat tac aac agg acg att aca ttc    672
   Leu Met Gly Ser Ser Asp Ser Asp Asn Tyr Asn Arg Thr Ile Thr Phe
       210                 215                 220
   cat cat aac tgg ttt gag aat ctg aat tcg cgt gtg ccg tca ttc cgt    720
   His His Asn Trp Phe Glu Asn Leu Asn Ser Arg Val Pro Ser Phe Arg
   225                 230                 235                 240
   ttc gga gaa ggc cat att tac aac aac tat ttc aat aaa atc atc gac    768
   Phe Gly Glu Gly His Ile Tyr Asn Asn Tyr Phe Asn Lys Ile Ile Asp
                   245                 250                 255
   agc gga att aat tcg agg atg ggc gcg cgc atc aga att gag aac aac    816
   Ser Gly Ile Asn Ser Arg Met Gly Ala Arg Ile Arg Ile Glu Asn Asn
               260                 265                 270
   ctc ttt gaa aac gcc aaa gat ccg att gtc tct tgg tac agc agt tca    864
   Leu Phe Glu Asn Ala Lys Asp Pro Ile Val Ser Trp Tyr Ser Ser Ser
           275                 280                 285
   ccg ggc tat tgg cat gta tcc aac aac aaa ttt gta aac tct agg ggc    912
   Pro Gly Tyr Trp His Val Ser Asn Asn Lys Phe Val Asn Ser Arg Gly
       290                 295                 300
   agt atg ccg act acc tct act aca acc tat aat ccg cca tac agc tac    960
   Ser Met Pro Thr Thr Ser Thr Thr Thr Tyr Asn Pro Pro Tyr Ser Tyr
   305                 310                 315                 320
   tca ctc gac aat gtc gac aat gta aaa tca atc gtc aag caa aat gcc    1008
   Ser Leu Asp Asn Val Asp Asn Val Lys Ser Ile Val Lys Gln Asn Ala
                   325                 330                 335
   gga gtc ggc aaa atc aat cca gct agc att gaa ggc aga cat cat aat    1056
   Gly Val Gly Lys Ile Asn Pro Ala Ser Ile Glu Gly Arg His His Asn
               340                 345                 350
   ggg aca aat ggg acg atg atg caa tac ttt gaa tgg cac ttg cct aat    1104
   Gly Thr Asn Gly Thr Met Met Gln Tyr Phe Glu Trp His Leu Pro Asn
           355                 360                 365
   gat ggg aat cac tgg aat aga tta aga gat gat gct agt aat cta aga    1152
   Asp Gly Asn His Trp Asn Arg Leu Arg Asp Asp Ala Ser Asn Leu Arg
       370                 375                 380
   aat aga ggt ata acc gct att tgg att ccg cct gcc tgg aaa ggg act    1200
   Asn Arg Gly Ile Thr Ala Ile Trp Ile Pro Pro Ala Trp Lys Gly Thr
   385                 390                 395                 400
   tcg caa aat gat gtg ggg tat gga gcc tat gat ctt tat gat tta ggg    1248
   Ser Gln Asn Asp Val Gly Tyr Gly Ala Tyr Asp Leu Tyr Asp Leu Gly
                   405                 410                 415
   gaa ttt aat caa aag ggg acg gtt cgt act aag tat ggg aca cgt agt    1296
   Glu Phe Asn Gln Lys Gly Thr Val Arg Thr Lys Tyr Gly Thr Arg Ser
               420                 425                 430
   caa ttg gag tct gcc atc cat gct tta aag aat aat ggc gtt caa gtt    1344
   Gln Leu Glu Ser Ala Ile His Ala Leu Lys Asn Asn Gly Val Gln Val
           435                 440                 445
   tat ggg gat gta gtg atg aac cat aaa gga gga gct gat gct aca gaa    1392
   Tyr Gly Asp Val Val Met Asn His Lys Gly Gly Ala Asp Ala Thr Glu
       450                 455                 460
   aac gtt ctt gct gtc gag gtg aat cca aat aac cgg aat caa gaa ata    1440
   Asn Val Leu Ala Val Glu Val Asn Pro Asn Asn Arg Asn Gln Glu Ile
   465                 470                 475                 480
   tct ggg gac tac aca att gag gct tgg act aag ttt gat ttt cca ggg    1488
   Ser Gly Asp Tyr Thr Ile Glu Ala Trp Thr Lys Phe Asp Phe Pro Gly
                   485                 490                 495
   agg ggt aat aca tac tca gac ttt aaa tgg cgt tgg tat cat ttc gat    1536
   Arg Gly Asn Thr Tyr Ser Asp Phe Lys Trp Arg Trp Tyr His Phe Asp
               500                 505                 510
   ggt gta gat tgg gat caa tca cga caa ttc caa aat cgt atc tac aaa    1584
   Gly Val Asp Trp Asp Gln Ser Arg Gln Phe Gln Asn Arg Ile Tyr Lys
           515                 520                 525
   ttc cga ggt gat ggt aag gca tgg gat tgg gaa gta gat tcg gaa aat    1632
   Phe Arg Gly Asp Gly Lys Ala Trp Asp Trp Glu Val Asp Ser Glu Asn
       530                 535                 540
   gga aat tat gat tat tta atg tat gca gat gta gat atg gat cat ccg    1680
   Gly Asn Tyr Asp Tyr Leu Met Tyr Ala Asp Val Asp Met Asp His Pro
   545                 550                 555                 560
   gag gta gta aat gag ctt aga aga tgg gga gaa tgg tat aca aat aca    1728
   Glu Val Val Asn Glu Leu Arg Arg Trp Gly Glu Trp Tyr Thr Asn Thr
                   565                 570                 575
   tta aat ctt gat gga ttt agg atc gat gcg gtg aag cat att aaa tat    1776
   Leu Asn Leu Asp Gly Phe Arg Ile Asp Ala Val Lys His Ile Lys Tyr
               580                 585                 590
   agc ttt aca cgt gat tgg ttg acc cat gta aga aac gca acg gga aaa    1824
   Ser Phe Thr Arg Asp Trp Leu Thr His Val Arg Asn Ala Thr Gly Lys
           595                 600                 605
   gaa atg ttt gct gtt gct gaa ttt tgg aaa aat gat tta ggt gcc ttg    1872
   Glu Met Phe Ala Val Ala Glu Phe Trp Lys Asn Asp Leu Gly Ala Leu
       610                 615                 620
   gag aac tat tta aat aaa aca aac tgg aat cat tct gtc ttt gat gtc    1920
   Glu Asn Tyr Leu Asn Lys Thr Asn Trp Asn His Ser Val Phe Asp Val
   625                 630                 635                 640
   ccc ctt cat tat aat ctt tat aac gcg tca aat agt gga ggc aac tat    1968
   Pro Leu His Tyr Asn Leu Tyr Asn Ala Ser Asn Ser Gly Gly Asn Tyr
                   645                 650                 655
   gac atg gca aaa ctt ctt aat gga acg gtt gtt caa aag cat cca atg    2016
   Asp Met Ala Lys Leu Leu Asn Gly Thr Val Val Gln Lys His Pro Met
               660                 665                 670
   cat gcc gta act ttt gtg gat aat cac gat tct caa cct ggg gaa tca    2064
   His Ala Val Thr Phe Val Asp Asn His Asp Ser Gln Pro Gly Glu Ser
           675                 680                 685
   tta gaa tca ttt gta caa gaa tgg ttt aag cca ctt gct tat gcg ctt    2112
   Leu Glu Ser Phe Val Gln Glu Trp Phe Lys Pro Leu Ala Tyr Ala Leu
       690                 695                 700
   att tta aca aga gaa caa ggc tat ccc tct gtc ttc tat ggt gac tac    2160
   Ile Leu Thr Arg Glu Gln Gly Tyr Pro Ser Val Phe Tyr Gly Asp Tyr
   705                 710                 715                 720
   tat gga att cca aca cat agt gtc cca gca atg aaa gcc aag att gat    2208
   Tyr Gly Ile Pro Thr His Ser Val Pro Ala Met Lys Ala Lys Ile Asp
                   725                 730                 735
   cca atc tta gag gcg cgt caa aat ttt gca tat gga aca caa cat gat     2256
   Pro Ile Leu Glu Ala Arg Gln Asn Phe Ala Tyr Gly Thr Gln His Asp
                   740                 745                 750
   tat ttt gac cat cat aat ata atc gga tgg aca cgt gaa gga aat acc     2304
   Tyr Phe Asp His His Asn Ile Ile Gly Trp Thr Arg Glu Gly Asn Thr
               755                 760                 765
   acg cat ccc aat tca gga ctt gcg act atc atg tcg gat ggg cca ggg     2352
   Thr His Pro Asn Ser Gly Leu Ala Thr Ile Met Ser Asp Gly Pro Gly
           770                 775                 780
   gga gag aaa tgg atg tac gta ggg caa aat aaa gca ggt caa gtt tgg     2400
   Gly Glu Lys Trp Met Tyr Val Gly Gln Asn Lys Ala Gly Gln Val Trp
   785                 790                 795                 800
   cat gac ata act gga aat aaa cca gga aca gtt acg atc aat gca gat     2448
   His Asp Ile Thr Gly Asn Lys Pro Gly Thr Val Thr Ile Asn Ala Asp
                   805                 810                 815
   gga tgg gct aat ttt tca gta aat gga gga tct gtt tcc att tgg gtg     2496
   Gly Trp Ala Asn Phe Ser Val Asn Gly Gly Ser Val Ser Ile Trp Val
                   820                 825                 830
   aaa cga                                                             2502
   Lys Arg
<210> 12
   <211> 834
   <212> PRT
   <213> Artificial Sequence
   <223> Description of Artificial Sequence:Details available in the text.
<400> 12
   Met Lys Gln Gln Lys Arg Leu Tyr Ala Arg Leu Leu Thr Leu Leu Phe
     1               5                  10                  15
   Ala Leu Ile Phe Leu Leu Pro His Ser Ala Ala Ala Ala Ala Ser Ala
                20                  25                  30
   Leu Asn Ser Gly Lys Val Asn Pro Leu Ala Asp Phe Ser Leu Lys Gly
            35                  40                  45
   Phe Ala Ala Leu Asn Gly Gly Thr Thr Gly Gly Glu Gly Gly Gln Thr
       50                  55                  60
   Val Thr Val Thr Thr Gly Asp Gln Leu Ile Ala Ala Leu Lys Asn Lys
   65 70 75 80
   Asn Ala Asn Thr Pro Leu Lys Ile Tyr Val Asn Gly Thr Ile Thr Thr
   85 90 95
   Ser Asn Thr Ser Ala Ser Lys Ile Asp Val Lys Asp Val Ser Asn Val
   100 105 110
   Ser Ile Val Gly Ser Gly Thr Lys Gly Glu Leu Lys Gly Ile Gly Ile
   115 120 125
   Lys Ile Trp Arg Ala Asn Asn Ile Ile Ile Arg Asn Leu Lys Ile His
   130 135 140
   Glu Val Ala Ser Gly Asp Lys Asp Ala Ile Gly Ile Glu Gly Pro Ser
   145 150 155 160
   Lys Asn Ile Trp Val Asp His Asn Glu Leu Tyr His Ser Leu Asn Val
   165 170 175
   Asp Lys Asp Tyr Tyr Asp Gly Leu Phe Asp Val Lys Arg Asp Ala Glu
   180 185 190
   Tyr Ile Thr Phe Ser Trp Asn Tyr Val His Asp Gly Trp Lys Ser Met
   195 200 205
   Leu Met Gly Ser Ser Asp Ser Asp Asn Tyr Asn Arg Thr Ile Thr Phe
   210 215 220
   His His Asn Trp Phe Glu Asn Leu Asn Ser Arg Val Pro Ser Phe Arg
   225 230 235 240
   Phe Gly Glu Gly His Ile Tyr Asn Asn Tyr Phe Asn Lys Ile Ile Asp
   245 250 255
   Ser Gly Ile Asn Ser Arg Met Gly Ala Arg Ile Arg Ile Glu Asn Asn
   260 265 270
   Leu Phe Glu Asn Ala Lys Asp Pro Ile Val Ser Trp Tyr Ser Ser Ser
   275 280 285
   Pro Gly Tyr Trp His Val Ser Asn Asn Lys Phe Val Asn Ser Arg Gly
   290 295 300
   Ser Met Pro Thr Thr Ser Thr Thr Thr Tyr Asn Pro Pro Tyr Ser Tyr
   305 310 315 320
   Ser Leu Asp Asn Val Asp Asn Val Lys Ser Ile Val Lys Gln Asn Ala
   325 330 335
   Gly Val Gly Lys Ile Asn Pro Ala Ser Ile Glu Gly Arg His His Asn
   340 345 350
   Gly Thr Asn Gly Thr Met Met Gln Tyr Phe Glu Trp His Leu Pro Asn
   355 360 365
   Asp Gly Asn His Trp Asn Arg Leu Arg Asp Asp Ala Ser Asn Leu Arg
   370 375 380
   Asn Arg Gly Ile Thr Ala Ile Trp Ile Pro Pro Ala Trp Lys Gly Thr
   385 390 395 400
   Ser Gln Asn Asp Val Gly Tyr Gly Ala Tyr Asp Leu Tyr Asp Leu Gly
   405 410 415
   Glu Phe Asn Gln Lys Gly Thr Val Arg Thr Lys Tyr Gly Thr Arg Ser
   420 425 430
   Gln Leu Glu Ser Ala Ile His Ala Leu Lys Asn Asn Gly Val Gln Val
   435 440 445
   Tyr Gly Asp Val Val Met Asn His Lys Gly Gly Ala Asp Ala Thr Glu
   450 455 460
   Asn Val Leu Ala Val Glu Val Asn Pro Asn Asn Arg Asn Gln Glu Ile
   465 470 475 480
   Ser Gly Asp Tyr Thr Ile Glu Ala Trp Thr Lys Phe Asp Phe Pro Gly
   485 490 495
   Arg Gly Asn Thr Tyr Ser Asp Phe Lys Trp Arg Trp Tyr His Phe Asp
   500 505 510
   Gly Val Asp Trp Asp Gln Ser Arg Gln Phe Gln Asn Arg Ile Tyr Lys
   515 520 525
   Phe Arg Gly Asp Gly Lys Ala Trp Asp Trp Glu Val Asp Ser Glu Asn
   530 535 540
   Gly Asn Tyr Asp Tyr Leu Met Tyr Ala Asp Val Asp Met Asp His Pro
   545 550 555 560
   Glu Val Val Asn Glu Leu Arg Arg Trp Gly Glu Trp Tyr Thr Asn Thr
   565 570 575
   Leu Asn Leu Asp Gly Phe Arg Ile Asp Ala Val Lys His Ile Lys Tyr
   580 585 590
   Ser Phe Thr Arg Asp Trp Leu Thr His Val Arg Asn Ala Thr Gly Lys
   595 600 605
   Glu Met Phe Ala Val Ala Glu Phe Trp Lys Asn Asp Leu Gly Ala Leu
   610 615 620
   Glu Asn Tyr Leu Asn Lys Thr Asn Trp Asn His Ser Val Phe Asp Val
   625 630 635 640
   Pro Leu His Tyr Asn Leu Tyr Asn Ala Ser Asn Ser Gly Gly Asn Tyr
   645 650 655
   Asp Met Ala Lys Leu Leu Asn Gly Thr Val Val Gln Lys His Pro Met
   660 665 670
   His Ala Val Thr Phe Val Asp Asn His Asp Ser Gln Pro Gly Glu Ser
   675 680 685
   Leu Glu Ser Phe Val Gln Glu Trp Phe Lys Pro Leu Ala Tyr Ala Leu
   690 695 700
   Ile Leu Thr Arg Glu Gln Gly Tyr Pro Ser Val Phe Tyr Gly Asp Tyr
   705 710 715 720
   Tyr Gly Ile Pro Thr His Ser Val Pro Ala Met Lys Ala Lys Ile Asp
   725 730 735
   Pro Ile Leu Glu Ala Arg Gln Asn Phe Ala Tyr Gly Thr Gln His Asp
   740 745 750
   Tyr Phe Asp His His Asn Ile Ile Gly Trp Thr Arg Glu Gly Asn Thr
   755 760 765
   Thr His Pro Asn Ser Gly Leu Ala Thr Ile Met Ser Asp Gly Pro Gly
   770 775 780
   Gly Glu Lys Trp Met Tyr Val Gly Gln Asn Lys Ala Gly Gln Val Trp
   785 790 795 800
   His Asp Ile Thr Gly Asn Lys Pro Gly Thr Val Thr Ile Asn Ala Asp
   805 810 815
   Gly Trp Ala Asn Phe Ser Val Asn Gly Gly Ser Val Ser Ile Trp Val
   820 825 830
   Lys Arg
<210> 13
   <211> 1134
   <212> DNA
   <213> Artificial Sequence
<220>
   <223> Description of Artificial Sequence:Details available in the text.
<220>
   <221> CDS
   <222> (1)..(1134)
<400> 13
   atg aaa caa caa aaa cgg ctt tac gcc cga ttg ctg acg ctg tta ttt      48
   Met Lys Gln Gln Lys Arg Leu Tyr Ala Arg Leu Leu Thr Leu Leu Phe
     1               5                  10                  15
   gcg ctc atc ttc ttg ctg cct cat tct gca gcc gcg gca gct tct gcc      96
   Ala Leu Ile Phe Leu Leu Pro His Ser Ala Ala Ala Ala Ala Ser Ala
                20                  25                  30
   tta aac tcg ggc aaa gta aat ccg ctt gcc gac ttc agc tta aaa ggc     144
   Leu Asn Ser Gly Lys Val Asn Pro Leu Ala Asp Phe Ser Leu Lys Gly
            35                  40                  45
   ttt gcc gca cta aac ggc gga aca acg ggc gga gaa ggc ggt cag acg     192
   Phe Ala Ala Leu Asn Gly Gly Thr Thr Gly Gly Glu Gly Gly Gln Thr
        50                  55                  60
   gta acc gta aca acg gga gat cag ctg att gcg gca tta aaa aat aag     240
   Val Thr Val Thr Thr Gly Asp Gln Leu Ile Ala Ala Leu Lys Asn Lys
    65                  70                  75                  80
   aat gca aat acg cct tta aaa att tat gtc aac ggc acc att aca aca     288
   Asn Ala Asn Thr Pro Leu Lys Ile Tyr Val Asn Gly Thr Ile Thr Thr
                    85                  90                  95
   tca aat aca tcc gca tca aag att gac gtc aaa gac gtg tca aac gta     336
   Ser Asn Thr Ser Ala Ser Lys Ile Asp Val Lys Asp Val Ser Asn Val
               100                 105                 110
   tcg att gtc gga tca ggg acc aaa ggg gaa ctc aaa ggg atc ggc atc     384
   Ser Ile Val Gly Ser Gly Thr Lys Gly Glu Leu Lys Gly Ile Gly Ile
           115                 120                 125
   aaa ata tgg cgg gcc aac aac atc atc atc cgc aac ttg aaa att cac     432
   Lys Ile Trp Arg Ala Asn Asn Ile Ile Ile Arg Asn Leu Lys Ile His
       130                 135                 140
   gag gtc gcc tca ggc gat aaa gac gcg atc ggc att gaa ggc cct tct     480
   Glu Val Ala Ser Gly Asp Lys Asp Ala Ile Gly Ile Glu Gly Pro Ser
   145                 150                 155                 160
   aaa aac att tgg gtt gat cat aat gag ctt tac cac agc ctg aac gtt     528
   Lys Asn Ile Trp Val Asp His Asn Glu Leu Tyr His Ser Leu Asn Val
                   165                 170                 175
   gac aaa gat tac tat gac gga tta ttt gac gtc aaa aga gat gcg gaa     576
   Asp Lys Asp Tyr Tyr Asp Gly Leu Phe Asp Val Lys Arg Asp Ala Glu
               180                 185                 190
   tat att aca ttc tct tgg aac tat gtg cac gat gga tgg aaa tca atg     624
   Tyr Ile Thr Phe Ser Trp Asn Tyr Val His Asp Gly Trp Lys Ser Met
           195                 200                 205
   ctg atg ggt tca tcg gac agc gat aat tac aac agg acg att aca ttc     672
   Leu Met Gly Ser Ser Asp Ser Asp Asn Tyr Asn Arg Thr Ile Thr Phe
       210                 215                 220
   cat cat aac tgg ttt gag aat ctg aat tcg cgt gtg ccg tca ttc cgt     720
   His His Asn Trp Phe Glu Asn Leu Asn Ser Arg Val Pro Ser Phe Arg
   225                 230                 235                 240
   ttc gga gaa ggc cat att tac aac aac tat ttc aat aaa atc atc gac     768
   Phe Gly Glu Gly His Ile Tyr Asn Asn Tyr Phe Asn Lys Ile Ile Asp
                   245                 250                 255
   agc gga att aat tcg agg atg ggc gcg cgc atc aga att gag aac aac     816
   Ser Gly Ile Asn Ser Arg Met Gly Ala Arg Ile Arg Ile Glu Asn Asn
               260                 265                 270
   ctc ttt gaa aac gcc aaa gat ccg att gtc tct tgg tac agc agt tca     864
   Leu Phe Glu Asn Ala Lys Asp Pro Ile Val Ser Trp Tyr Ser Ser Ser
           275                 280                 285
   ccg ggc tat tgg cat gta tcc aac aac aaa ttt gta aac tct agg ggc     912
   Pro Gly Tyr Trp His Val Ser Asn Asn Lys Phe Val Asn Ser Arg Gly
       290                 295                 300
   agt atg ccg act acc tct act aca acc tat aat ccg cca tac agc tac     960
   Ser Met Pro Thr Thr Ser Thr Thr Thr Tyr Asn Pro Pro Tyr Ser Tyr
   305 310 315 320
   tca ctc gac aat gtc gac aat gta aaa tca atc gtc aag caa aat gcc 1008
   Ser Leu Asp Asn Val Asp Asn Val Lys Ser Ile Val Lys Gln Asn Ala
   325 330 335
   gga gtc ggc aaa atc aat cca gct agc aaa aga cat gcc gaa gga aca 1056
   Gly Val Gly Lys Ile Asn Pro Ala Ser Lys Arg His Ala Glu Gly Thr
   340 345 350
   ttt acg tca gac gtc tca tca tat tta gaa ggc cag gca gcc aaa gaa 1104
   Phe Thr Ser Asp Val Ser Ser Tyr Leu Glu Gly Gln Ala Ala Lys Glu
   355 360 365
   ttc atc gca tgg tta gtc aaa ggc agg gga 1134
   Phe Ile Ala Trp Leu Val Lys Gly Arg Gly
   370 375
<210> 14
   <211> 378
   **<212>** PRT
   **<213>** Artificial Sequence
   **<223>** Description of Artificial Sequence:Details available in the text.
<400> 14
   Met Lys Gln Gln Lys Arg Leu Tyr Ala Arg Leu Leu Thr Leu Leu Phe
   1 5 10 15
   Ala Leu Ile Phe Leu Leu Pro His Ser Ala Ala Ala Ala Ala Ser Ala
   20 25 30
   Leu Asn Ser Gly Lys Val Asn Pro Leu Ala Asp Phe Ser Leu Lys Gly
   35 40 45
   Phe Ala Ala Leu Asn Gly Gly Thr Thr Gly Gly Glu Gly Gly Gln Thr
   50 55 60
   Val Thr Val Thr Thr Gly Asp Gln Leu Ile Ala Ala Leu Lys Asn Lys
   65 70 75 80
   Asn Ala Asn Thr Pro Leu Lys Ile Tyr Val Asn Gly Thr Ile Thr Thr
   85 90 95
   Ser Asn Thr Ser Ala Ser Lys Ile Asp Val Lys Asp Val Ser Asn Val
   100 105 110
   Ser Ile Val Gly Ser Gly Thr Lys Gly Glu Leu Lys Gly Ile Gly Ile
   115 120 125
   Lys Ile Trp Arg Ala Asn Asn Ile Ile Ile Arg Asn Leu Lys Ile His
   130 135 140
   Glu Val Ala Ser Gly Asp Lys Asp Ala Ile Gly Ile Glu Gly Pro Ser
   145 150 155 160
   Lys Asn Ile Trp Val Asp His Asn Glu Leu Tyr His Ser Leu Asn Val
   165 170 175
   Asp Lys Asp Tyr Tyr Asp Gly Leu Phe Asp Val Lys Arg Asp Ala Glu
   180 185 190
   Tyr Ile Thr Phe Ser Trp Asn Tyr Val His Asp Gly Trp Lys Ser Met
   195 200 205
   Leu Met Gly Ser Ser Asp Ser Asp Asn Tyr Asn Arg Thr Ile Thr Phe
   210 215 220
   His His Asn Trp Phe Glu Asn Leu Asn Ser Arg Val Pro Ser Phe Arg
   225 230 235 240
   Phe Gly Glu Gly His Ile Tyr Asn Asn Tyr Phe Asn Lys Ile Ile Asp
   245 250 255
   Ser Gly Ile Asn Ser Arg Met Gly Ala Arg Ile Arg Ile Glu Asn Asn
   260 265 270
   Leu Phe Glu Asn Ala Lys Asp Pro Ile Val Ser Trp Tyr Ser Ser Ser
   275 280 285
   Pro Gly Tyr Trp His Val Ser Asn Asn Lys Phe Val Asn Ser Arg Gly
   290 295 300
   Ser Met Pro Thr Thr Ser Thr Thr Thr Tyr Asn Pro Pro Tyr Ser Tyr
   305 310 315 320
   Ser Leu Asp Asn Val Asp Asn Val Lys Ser Ile Val Lys Gln Asn Ala
   325 330 335
   Gly Val Gly Lys Ile Asn Pro Ala Ser Lys Arg His Ala Glu Gly Thr
   340 345 350
   Phe Thr Ser Asp Val Ser Ser Tyr Leu Glu Gly Gln Ala Ala Lys Glu
   355 360 365
   Phe Ile Ala Trp Leu Val Lys Gly Arg Gly
   370 375
<210> 15
   <211> 1158
   <212> DNA
   <213> Artificial Sequence
<220>
   <223> Description of Artificial Sequence: Details available in the text.
<220>
   <221> CDS
   <222> (1)..(1158)
<400> 15
   atg aaa caa caa aaa cgg ctt tac gcc cga ttg ctg acg ctg tta ttt   48
   Met Lys Gln Gln Lys Arg Leu Tyr Ala Arg Leu Leu Thr Leu Leu Phe
     1               5                  10                  15
   gcg ctc atc ttc ttg ctg cct cat tct gca gcc gcg gca gct tct gcc   96
   Ala Leu Ile Phe Leu Leu Pro His Ser Ala Ala Ala Ala Ala Ser Ala
                20                  25                  30
   tta aac tcg ggc aaa gta aat ccg ctt gcc gac ttc agc tta aaa ggc   144
   Leu Asn Ser Gly Lys Val Asn Pro Leu Ala Asp Phe Ser Leu Lys Gly
            35                  40                  45
   ttt gcc gca cta aac ggc gga aca acg ggc gga gaa ggc ggt cag acg   192
   Phe Ala Ala Leu Asn Gly Gly Thr Thr Gly Gly Glu Gly Gly Gln Thr
        50                  55                  60
   gta acc gta aca acg gga gat cag ctg att gcg gca tta aaa aat aag   240
   Val Thr Val Thr Thr Gly Asp Gln Leu Ile Ala Ala Leu Lys Asn Lys
    65                  70                  75                  80
   aat gca aat acg cct tta aaa att tat gtc aac ggc acc att aca aca   288
   Asn Ala Asn Thr Pro Leu Lys Ile Tyr Val Asn Gly Thr Ile Thr Thr
                    85                  90                  95
   tca aat aca tcc gca tca aag att gac gtc aaa gac gtg tca aac gta   336
   Ser Asn Thr Ser Ala Ser Lys Ile Asp Val Lys Asp Val Ser Asn Val
               100                 105                 110
   tcg att gtc gga tca ggg acc aaa ggg gaa ctc aaa ggg atc ggc atc    384
   Ser Ile Val Gly Ser Gly Thr Lys Gly Glu Leu Lys Gly Ile Gly Ile
           115                 120                 125
   aaa ata tgg cgg gcc aac aac atc atc atc cgc aac ttg aaa att cac    432
   Lys Ile Trp Arg Ala Asn Asn Ile Ile Ile Arg Asn Leu Lys Ile His
       130                 135                 140
   gag gtc gcc tca ggc gat aaa gac gcg atc ggc att gaa ggc cct tct    480
   Glu Val Ala Ser Gly Asp Lys Asp Ala Ile Gly Ile Glu Gly Pro Ser
   145                 150                 155                 160
   aaa aac att tgg gtt gat cat aat gag ctt tac cac agc ctg aac gtt    528
   Lys Asn Ile Trp Val Asp His Asn Glu Leu Tyr His Ser Leu Asn Val
                   165                 170                 175
   gac aaa gat tac tat gac gga tta ttt gac gtc aaa aga gat gcg gaa    576
   Asp Lys Asp Tyr Tyr Asp Gly Leu Phe Asp Val Lys Arg Asp Ala Glu
               180                 185                 190
   tat att aca ttc tct tgg aac tat gtg cac gat gga tgg aaa tca atg    624
   Tyr Ile Thr Phe Ser Trp Asn Tyr Val His Asp Gly Trp Lys Ser Met
           195                 200                 205
   ctg atg ggt tca tcg gac agc gat aat tac aac agg acg att aca ttc    672
   Leu Met Gly Ser Ser Asp Ser Asp Asn Tyr Asn Arg Thr Ile Thr Phe
       210                 215                 220
   cat cat aac tgg ttt gag aat ctg aat tcg cgt gtg ccg tca ttc cgt    720
   His His Asn Trp Phe Glu Asn Leu Asn Ser Arg Val Pro Ser Phe Arg
   225                 230                 235                 240
   ttc gga gaa ggc cat att tac aac aac tat ttc aat aaa atc atc gac    768
   Phe Gly Glu Gly His Ile Tyr Asn Asn Tyr Phe Asn Lys Ile Ile Asp
                   245                 250                 255
   agc gga att aat tcg agg atg ggc gcg cgc atc aga att gag aac aac    816
   Ser Gly Ile Asn Ser Arg Met Gly Ala Arg Ile Arg Ile Glu Asn Asn
               260                 265                 270
   ctc ttt gaa aac gcc aaa gat ccg att gtc tct tgg tac agc agt tca    864
   Leu Phe Glu Asn Ala Lys Asp Pro Ile Val Ser Trp Tyr Ser Ser Ser
           275                 280                 285
   ccg ggc tat tgg cat gta tcc aac aac aaa ttt gta aac tct agg ggc    912
   Pro Gly Tyr Trp His Val Ser Asn Asn Lys Phe Val Asn Ser Arg Gly
       290                 295                 300
   agt atg ccg act acc tct act aca acc tat aat ccg cca tac agc tac    960
   Ser Met Pro Thr Thr Ser Thr Thr Thr Tyr Asn Pro Pro Tyr Ser Tyr
   305                 310                 315                 320
   tca ctc gac aat gtc gac aat gta aaa tca atc gtc aag caa aat gcc    1008
   Ser Leu Asp Asn Val Asp Asn Val Lys Ser Ile Val Lys Gln Asn Ala
                   325                 330                 335
   gga gtc ggc aaa atc aat cca gct agc cca gaa cca aca cct gag ccc    1056
   Gly Val Gly Lys Ile Asn Pro Ala Ser Pro Glu Pro Thr Pro Glu Pro
               340                 345                 350
   aca aaa aga cat gcc gaa gga aca ttt acg tca gac gtc tca tca tat    1104
   Thr Lys Arg His Ala Glu Gly Thr Phe Thr Ser Asp Val Ser Ser Tyr
           355                 360                 365
   tta gaa ggc cag gca gcc aaa gaa ttc atc gca tgg tta gtc aaa ggc    1152
   Leu Glu Gly Gln Ala Ala Lys Glu Phe Ile Ala Trp Leu Val Lys Gly
       370                 375                 380
   agg gga                                                            1158
   Arg Gly
   385
<210> 16
   <211> 386
   <212> PRT
   <213> Artificial Sequence
   <223> Description of Artificial Sequence: Details available in the text.
<400> 16
   Met Lys Gln Gln Lys Arg Leu Tyr Ala Arg Leu Leu Thr Leu Leu Phe
     1               5                  10                  15
   Ala Leu Ile Phe Leu Leu Pro His Ser Ala Ala Ala Ala Ala Ser Ala
                20                  25                  30
   Leu Asn Ser Gly Lys Val Asn Pro Leu Ala Asp Phe Ser Leu Lys Gly
            35                  40                  45
   Phe Ala Ala Leu Asn Gly Gly Thr Thr Gly Gly Glu Gly Gly Gln Thr
        50                  55                  60
   Val Thr Val Thr Thr Gly Asp Gln Leu Ile Ala Ala Leu Lys Asn Lys
    65                  70                  75                  80
   Asn Ala Asn Thr Pro Leu Lys Ile Tyr Val Asn Gly Thr Ile Thr Thr
   85 90 95
   Ser Asn Thr Ser Ala Ser Lys Ile Asp Val Lys Asp Val Ser Asn Val
   100 105 110
   Ser Ile Val Gly Ser Gly Thr Lys Gly Glu Leu Lys Gly Ile Gly Ile
   115 120 125
   Lys Ile Trp Arg Ala Asn Asn Ile Ile Ile Arg Asn Leu Lys Ile His
   130 135 140
   Glu Val Ala Ser Gly Asp Lys Asp Ala Ile Gly Ile Glu Gly Pro Ser
   145 150 155 160
   Lys Asn Ile Trp Val Asp His Asn Glu Leu Tyr His Ser Leu Asn Val
   165 170 175
   Asp Lys Asp Tyr Tyr Asp Gly Leu Phe Asp Val Lys Arg Asp Ala Glu
   180 185 190
   Tyr Ile Thr Phe Ser Trp Asn Tyr Val His Asp Gly Trp Lys Ser Met
   195 200 205
   Leu Met Gly Ser Ser Asp Ser Asp Asn Tyr Asn Arg Thr Ile Thr Phe
   210 215 220
   His His Asn Trp Phe Glu Asn Leu Asn Ser Arg Val Pro Ser Phe Arg
   225 230 235 240
   Phe Gly Glu Gly His Ile Tyr Asn Asn Tyr Phe Asn Lys Ile Ile Asp
   245 250 255
   Ser Gly Ile Asn Ser Arg Met Gly Ala Arg Ile Arg Ile Glu Asn Asn
   260 265 270
   Leu Phe Glu Asn Ala Lys Asp Pro Ile Val Ser Trp Tyr Ser Ser Ser
   275 280 285
   Pro Gly Tyr Trp His Val Ser Asn Asn Lys Phe Val Asn Ser Arg Gly
   290 295 300
   Ser Met Pro Thr Thr Ser Thr Thr Thr Tyr Asn Pro Pro Tyr Ser Tyr
   305 310 315 320
   Ser Leu Asp Asn Val Asp Asn Val Lys Ser Ile Val Lys Gln Asn Ala
   325 330 335
   Gly Val Gly Lys Ile Asn Pro Ala Ser Pro Glu Pro Thr Pro Glu Pro
   340 345 350
   Thr Lys Arg His Ala Glu Gly Thr Phe Thr Ser Asp Val Ser Ser Tyr
   355 360 365
   Leu Glu Gly Gln Ala Ala Lys Glu Phe Ile Ala Trp Leu Val Lys Gly
   370 375 380
   Arg Gly
   385
<210> 17
   <211> 160
   <212> DNA
   <213> Homo sapiens
<220>
   <221> CDS
   <222> (1) .. (159)
<400> 17
   ttc gtc aac cag cat tta tgt ggc tca cat ctg gta gag gcc ctg tat 48
   Phe Val Asn Gln His Leu Cys Gly Ser His Leu Val Glu Ala Leu Tyr
   1 5 10 15
   tta gtc tgt gga gag agg gga ttc ttt tat aca ccg aaa gcc gcg aaa 96
   Leu Val Cys Gly Glu Arg Gly Phe Phe Tyr Thr Pro Lys Ala Ala Lys
   20 25 30
   ggc att gtt gaa cag tgt tgc aca tca atc tgt agc tta tat cag ctg 144
   Gly Ile Val Glu Gln Cys Cys Thr Ser Ile Cys Ser Leu Tyr Gln Leu
   35 40 45
   gaa aat tac tgc aac t 160
   Glu Asn Tyr Cys Asn
   50
<210> 18
   <211> 53
   <212> PRT
   <213> Homo sapiens
<400> 18
   Phe Val Asn Gln His Leu Cys Gly Ser His Leu Val Glu Ala Leu Tyr
                   1               5                   10                  15
   Leu Val Cys Gly Glu Arg Gly Phe Phe Tyr Thr Pro Lys Ala Ala Lys
                       20                  25                  30
   Gly Ile Val Glu Gln Cys Cys Thr Ser Ile Cys Ser Leu Tyr Gln Leu
                   35                  40                  45
   Glu Asn Tyr Cys Asn
               50
<210> 19
   <211> 1227
   <212> DNA
   <213> Artificial Sequence
<220>
   <223> Description of Artificial Sequence: Details available in the text.
<220>
   <221> CDS
   <222> (1)..(1227)
<400> 19
   atg aaa caa caa aaa cgg ctt tac gcc cga ttg ctg acg ctg tta ttt    48
   Met Lys Gln Gln Lys Arg Leu Tyr Ala Arg Leu Leu Thr Leu Leu Phe
   1               5                   10                  15
   gcg ctc atc ttc ttg ctg cct cat tct gca gcc gcg gca gct tct gcc    96
   Ala Leu Ile Phe Leu Leu Pro His Ser Ala Ala Ala Ala Ala Ser Ala
               20                  25                  30
   tta aac tcg ggc aaa gta aat ccg ctt gcc gac ttc agc tta aaa ggc    144
   Leu Asn Ser Gly Lys Val Asn Pro Leu Ala Asp Phe Ser Leu Lys Gly
           35                  40                  45
   ttt gcc gca cta aac ggc gga aca acg ggc gga gaa ggc ggt cag acg    192
   Phe Ala Ala Leu Asn Gly Gly Thr Thr Gly Gly Glu Gly Gly Gln Thr
       50                  55                  60
   gta acc gta aca acg gga gat cag ctg att gcg gca tta aaa aat aag    240
   Val Thr Val Thr Thr Gly Asp Gln Leu Ile Ala Ala Leu Lys Asn Lys
   65                  70                  75                  80
   aat gca aat acg cct tta aaa att tat gtc aac ggc acc att aca aca    288
   Asn Ala Asn Thr Pro Leu Lys Ile Tyr Val Asn Gly Thr Ile Thr Thr
                   85                  90                  95
   tca aat aca tcc gca tca aag att gac gtc aaa gac gtg tca aac gta    336
   Ser Asn Thr Ser Ala Ser Lys Ile Asp Val Lys Asp Val Ser Asn Val
               100                 105                 110
   tcg att gtc gga tca ggg acc aaa ggg gaa ctc aaa ggg atc ggc atc    384
   Ser Ile Val Gly Ser Gly Thr Lys Gly Glu Leu Lys Gly Ile Gly Ile
           115                 120                 125
   aaa ata tgg cgg gcc aac aac atc atc atc cgc aac ttg aaa att cac    432
   Lys Ile Trp Arg Ala Asn Asn Ile Ile Ile Arg Asn Leu Lys Ile His
       130                 135                 140
   gag gtc gcc tca ggc gat aaa gac gcg atc ggc att gaa ggc cct tct    480
   Glu Val Ala Ser Gly Asp Lys Asp Ala Ile Gly Ile Glu Gly Pro Ser
   145                 150                 155                 160
   aaa aac att tgg gtt gat cat aat gag ctt tac cac agc ctg aac gtt    528
   Lys Asn Ile Trp Val Asp His Asn Glu Leu Tyr His Ser Leu Asn Val
                   165                 170                 175
   gac aaa gat tac tat gac gga tta ttt gac gtc aaa aga gat gcg gaa    576
   Asp Lys Asp Tyr Tyr Asp Gly Leu Phe Asp Val Lys Arg Asp Ala Glu
               180                 185                 190
   tat att aca ttc tct tgg aac tat gtg cac gat gga tgg aaa tca atg    624
   Tyr Ile Thr Phe Ser Trp Asn Tyr Val His Asp Gly Trp Lys Ser Met
           195                 200                 205
   ctg atg ggt tca tcg gac agc gat aat tac aac agg acg att aca ttc    672
   Leu Met Gly Ser Ser Asp Ser Asp Asn Tyr Asn Arg Thr Ile Thr Phe
       210                 215                 220
   cat cat aac tgg ttt gag aat ctg aat tcg cgt gtg ccg tca ttc cgt    720
   His His Asn Trp Phe Glu Asn Leu Asn Ser Arg Val Pro Ser Phe Arg
   225                 230                 235                 240
   ttc gga gaa ggc cat att tac aac aac tat ttc aat aaa atc atc gac    768
   Phe Gly Glu Gly His Ile Tyr Asn Asn Tyr Phe Asn Lys Ile Ile Asp
                   245                 250                 255
   agc gga att aat tcg agg atg ggc gcg cgc atc aga att gag aac aac    816
   Ser Gly Ile Asn Ser Arg Met Gly Ala Arg Ile Arg Ile Glu Asn Asn
               260                 265                 270
   ctc ttt gaa aac gcc aaa gat ccg att gtc tct tgg tac agc agt tca    864
   Leu Phe Glu Asn Ala Lys Asp Pro Ile Val Ser Trp Tyr Ser Ser Ser
           275                 280                 285
   ccg ggc tat tgg cat gta tcc aac aac aaa ttt gta aac tct agg ggc    912
   Pro Gly Tyr Trp His Val Ser Asn Asn Lys Phe Val Asn Ser Arg Gly
       290                 295                 300
   agt atg ccg act acc tct act aca acc tat aat ccg cca tac agc tac    960
   Ser Met Pro Thr Thr Ser Thr Thr Thr Tyr Asn Pro Pro Tyr Ser Tyr
   305                 310                 315                 320
   tca ctc gac aat gtc gac aat gta aaa tca atc gtc aag caa aat gcc    1008
   Ser Leu Asp Asn Val Asp Asn Val Lys Ser Ile Val Lys Gln Asn Ala
                   325                 330                 335
   gga gtc ggc aaa atc aat cca gct agc ccg gaa cca aca cca gag ccg    1056
   Gly Val Gly Lys Ile Asn Pro Ala Ser Pro Glu Pro Thr Pro Glu Pro
               340                 345                 350
   acc aaa agg ttc gtc aac cag cat tta tgt ggc tca cat ctg gta gag    1104
   Thr Lys Arg Phe Val Asn Gln His Leu Cys Gly Ser His Leu Val Glu
           355                 360                 365
   gcc ctg tat tta gtc tgt gga gag agg gga ttc ttt tat aca ccg aaa    1152
   Ala Leu Tyr Leu Val Cys Gly Glu Arg Gly Phe Phe Tyr Thr Pro Lys
       370                 375                 380
   gcc gcg aaa ggc att gtt gaa cag tgt tgc aca tca atc tgt agc tta    1200
   Ala Ala Lys Gly Ile Val Glu Gln Cys Cys Thr Ser Ile Cys Ser Leu
   385                 390                 395                 400
   tat cag ctg gaa aat tac tgc aac taa                                1227
   Tyr Gln Leu Glu Asn Tyr Cys Asn
                   405
<210> 20
   <211> 408
   <212> PRT
   <213> Artificial Sequence
   <223> Description of Artificial Sequence: Details available in the text.
<210> 21
   <211> 42
   <212> DNA
   <213> Artificial Sequence
<220>
   <223> Description of Artificial Sequence: Primer # LWN5494
<400> 21
   gtcgccgggg cggccgctat caattggtaa ctgtatctca gc 42
<210> 22
   <211> 64
   <212> DNA
   <213> Artificial Sequence
<220>
   <223> Description of Artificial Sequence: Primer # LWN5495
<400> 22
   gtcgcccggg agctctgatc aggtaccaag cttgtcgacc tgcagaatga ggcagcaaga 60
   agat 64
<210> 23
   <211> 61
   <212> DNA
   <213> Artificial Sequence
<220>
   <223> Description of Artificial Sequence: Primer #LWN5938
<400> 23
   gtcggcggcc gctgatcacg taccaagctt gtcgacctgc agaatgaggc agcaagaaga 60
   t 61
<210> 24
   <211> 35
   <212> DNA
   <213> Artificial Sequence
   <220>
   <223> Description of Artificial Sequence: Primer #LWN5939
<400> 24
   gtcggagctc tatcaattgg taactgtatc tcagc 35
<210> 25
   <211> 35
   <212> DNA
   <213> Artificial Sequence
<220>
   <223> Description of Artificial Sequence: Primer #LWN7864
<400> 25
   aacagctgat cacgactgat cttttagctt ggcac 35
<210> 26
   <211> 37
   <212> DNA
   **<213>** Artificial Sequence
<220>
   **<223>** Description of Artificial Sequence: Primer #LWN7901
<400> 26
   aactgcagcc gcggcacatc ataatgggac aaatggg 37
<210> 27
   <211> 39
   <212> DNA
   <213> Artificial Sequence
<220>
   <223> Description of Artificial Sequence: Primer Pecl.B.lich.upper.SacII
<400> 27
   ctaactgcag ccgcggcagc ttctgcctta aactcgggc 39
<210> 28
   <211> 42
   <212> DNA
   <213> Artificial Sequence
<220>
   <223> Description of Artificial Sequence: Primer Pecl.B.lich.lower.NotI
<400> 28
   gcgttgagac gcgcggccgc tgaatgcccc ggacgtttca cc 42
<210> 29
   <211> 105
   <212> DNA
   **<213>** Artificial Sequence
<220>
   **<223>** Description of Artificial Sequence: Primer 145424.forward.NheI
<400> 29
   gacaatgtcg acaatgtaaa atcaatcgtc aagcaaaatg ccggagtcgg caaaatcaat 60
   ccagctagca ttgaaggcag acatcataat gggacaaatg ggacg 105
<210> 30
   <211> 22
   <212> DNA
   <213> Artificial Sequence
<220>
   <223> Description of Artificial Sequence: Primer 101450.reverse
<400> 30
   catggtgaac caaagtgaaa cc 22
<210> 31
   <211> 78
   <212> DNA
   <213> Artificial Sequence
<220>
   <223> Description of Artificial Sequence: Primer 149217.forward.Nhel
<400> 31
   tgtttgctag caaaagacat gccgaaggaa catttacgtc agacgtctca tcatatttag 60
   aaggccaggc agccaaag 78
<210> 32
   <211> 70
   <212> DNA
   <213> Artificial Sequence
<220>
   <223> Description of Artificial Sequence: Primer 149216.reverae.EagI
<400> 32
   gcaaacggcc gaaagcttat cccctgcctt tgactaacca tgcgatgaat tctttggctg 60
   cctggccttc 70
<210> 33
   <211> 60
   <212> DNA
   <213> Artificial Sequence
<220>
   <223> Description of Artificial Sequence: Primer 159639.forward.NheI
<400> 33
   ccagctagcc cagaaccaac acctgagccc acaaaaagac atgccgaagg aacatttacg 60
<210> 34
   <211> 22
   <212> DNA
   <213> Artificial Sequence
<220>
   <223> Description of Artificial Sequence: Primer 101450.reverse
<400> 34
   catggtgaac caaagtgaaa cc 22
<210> 35
   <211> 36
   <212> DNA
   <213> Artificial Sequence
<220>
   <223> Description of Artificial Sequence: Primer B5456H02.reverse
<400> 35
   ggtgttggtt ctgggctagc tggattgatt ttgccg 36
<210> 36
   <211> 22
   <212> DNA
   <213> Artificial Sequence
<220>
   <223> Description of Artificial Sequence: Primer 142670.forward
<400> 36
   cagcgataat tacaacagga cg 22
<210> 37
   <211> 126
   <212> DNA
   <213> Artificial Sequence
<220>
   <223> Description of Artificial Sequence: Primer Pecl.ISFUS.NheI.upper (#149171)
<400> 37
   catcatgcta gcccggaacc aacaccagag ccgaccaaaa ggttcgtcaa ccagcattta 60
   tgtggctcac atctggtaga ggccctgtat ttagtctgtg gagagagggg attcttttat 120
   acaccg                                                            126
<210> 38
   <211> 120
   <212> DNA
   <213> Artificial Sequence
<220>
   <223> Description of Artificial Sequence: Primer Peel.ISFUS.NotI.Lower (#149172)
<400> 38
   gcgttgagac gcggccgctt agttgcagta attttccagc tgatataagc tacagattga 60
   tgtgcaacac tgttcaacaa tgcctttcgc ggctttcggt gtataaaaga atcccctctc 120

## Description

The present invention relates to microbial production of a polypeptide by means of the expression of a fusion protein. More specifically, the invention relates to a cell for improved production of a fusion protein comprising a native pectate lyase fused to an exogenous polypeptide and to a process for producing the fusion protein and/or the polypeptide.

### BACKGROUND OF THE INVENTION

Proteins or polypeptides are currently being produced industrially in a number of ways, all involving cultivation of a microorganism which produces the protein. Several methods for optimizing industrial protein yield are known in the art and are being utilized routinely such as manipulations of the growth media, alterations of the microorganism e.g. by mutations, variations of the genetic elements like promoters and signal sequences etc. that influence expression of the gene encoding the desired protein, and also manipulations of the gene itself in order to e.g. enhance protein stability or activity.

Fusion-proteins have previously been described as a way of producing proteins that are otherwise difficult to obtain, one example could be active Human antibodies (Goshorn, S.C. et al., Cancer Research, (1993) Vol. 53 (9) pp. 2123-2127).

The object of the invention is to provide a microbial method for producing a polypeptide in high yields.

### SUMMARY OF THE INVENTION

The inventors have identified a *Bacillus* pectate lyase which can be produced in a high yield and is encoded by an isolated first DNA segment which DNA segment can be fused into one open reading frame with a second DNA segment encoding a polypeptide of exogenous origin, the fusion resulting in a DNA sequence encoding a fusion protein which can be produced in high yields under suitable conditions.

Further, the inventors have found that certain amino acid sequences can serve as target sites for proteolytic cleavage when introduced between the pectate lyase and the exogenous polypeptide.

Accordingly, in a first aspect the invention relates to a cell comprising a DNA sequence coding for at least the following elements fused sequentially into one open reading frame, a pectate lyase, a target site for proteolytic cleavage, and a polypeptide of exogenous origin.

The inventors have also succeeded in including an amino acid linker between the pectate lyase and the exogenous polypeptide which under certain circumstances increases the stability of the fusion protein.

Accordingly, in a second aspect the invention relates to a cell comprising a DNA sequence coding for at least the following elements fused sequentially into one open reading frame, a pectate lyase, a linker of at least two amino acids, a target site for proteolytic cleavage, and a polypeptide of exogenous origin

In a third aspect, the present invention relates to a method for producing a protein, the method comprising the steps of:
i) constructing a suitable cell comprising a DNA sequence coding for at least the following sequential elements fused into one open reading frame (ORF), a pectate lyase, target site for proteolytic cleavage, and a polypeptide of exogenous origin,
ii) culturing the cell constructed in step (i) under suitable conditions for growth and secretion,
iii) recovering the protein, and
iv) optionally cleaving the protein at the target site.

By using the cell or the method of this invention it is possible to produce desirable exogeneous polypeptides such as hormones, functional hormone analogues, enzymes and artificial polypeptides in high yields, thus improving the production economy or, for certain polypeptides, even making the practical exploitation of such desirable polypeptides economically feasible.

### BRIEF DESCRIPTION OF THE FIGURES

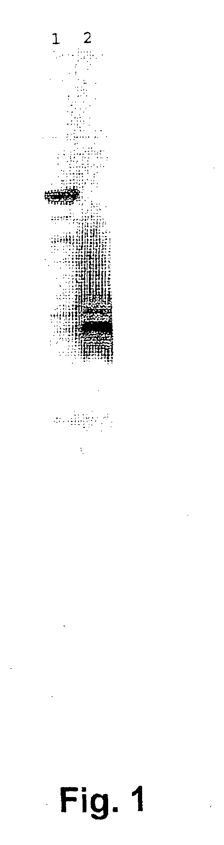

In the appended figures
Figure 1 shows a Western blot exemplifying the expression of the fusion polypeptide Pectate lyase-ASKR-GLP1 (7-37) according to example 2. Legends to the figure: Lane 1: Bacillus culture of Pectate lyase-ASKR-GLP1(7-37); Lane 2: GLP1 standard 100 mg/l;

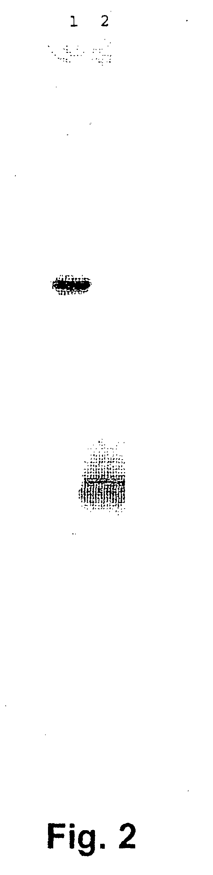

Figure 2 shows a Western blot exemplifying the expression of the fusion polypeptide Pectate lyase-ASKR-GLP1 (7-37) according to example 2. Legends to figure 2: Lane 1: Purified Pectate lyase-ASKR-GLP1(7-37) diluted 10x. Lane 2: Mixed standard of MI3(12,5 mg/l) and GLP1(25 mg/l);

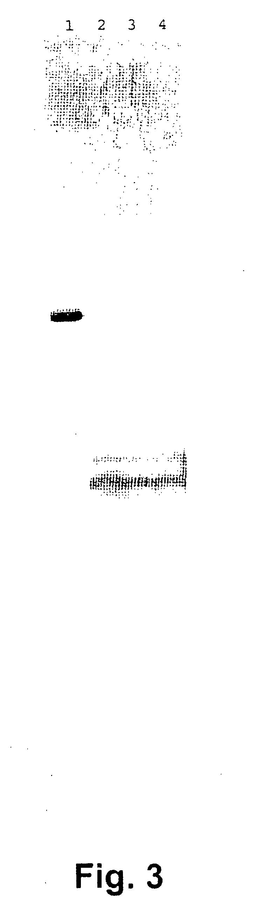

Figure 3 shows a Western blot exemplifying the expression by a Bacillus subtilis culture of the fusion polypeptide Pectate lyase-AS-PEPTPEPTKR-GLP1 (7-37) according to example 3. Legends to figure 3: Lane 1: culture (control); Lane 2: GLP1 standard (100 mg/l); Lane 3: GLP1 standard (50 mg/l); Lane 4: GLP1 standard (25 mg/l); and

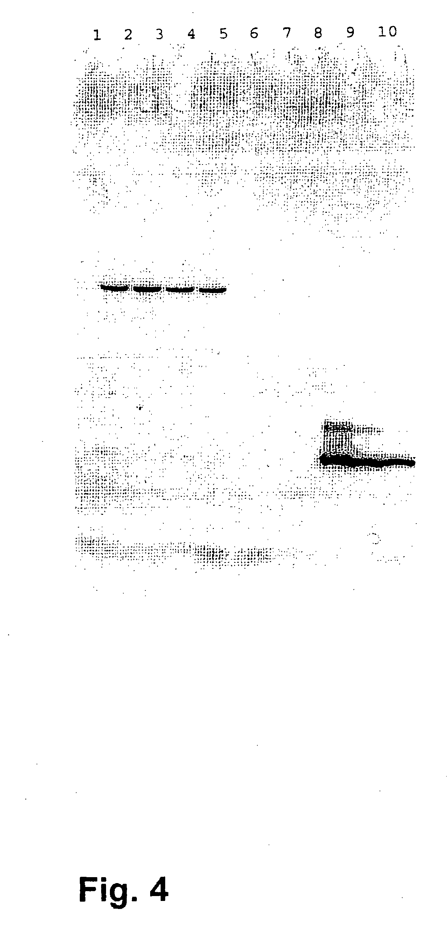

Figure 4 shows a Western blot exemplifying the expression of the fusion polypeptide Pectate lyase-AS-PEPTPEPTKR-MI3 according to example 4. Legends to figure 4: Lane 1: MB1009-1(Pectate lyase-AS-PEPTPEPTKR-MI3). Lane 2: MB1009-1(Pectate lyase-AS-PEPTPEPTKR-MI3). Lane 3: MB1009-4(Pectate lyase-AS-PEPTPEPTKR-MI3). Lane 4: MB1009-4(Pectate lyase-AS-PEPTPEPTKR-MI3); Lane 5: MB1009-7(Negative control); Lane 6: MB1009-7(Negative control); Lane 7: MB1009-7(Negative control); Lane 8: Standard MI3, 50 mg/l; Lane 9: Standard MI3, 25 mg/l; Lane 10: Standard MI3, 12,5 mg/l.

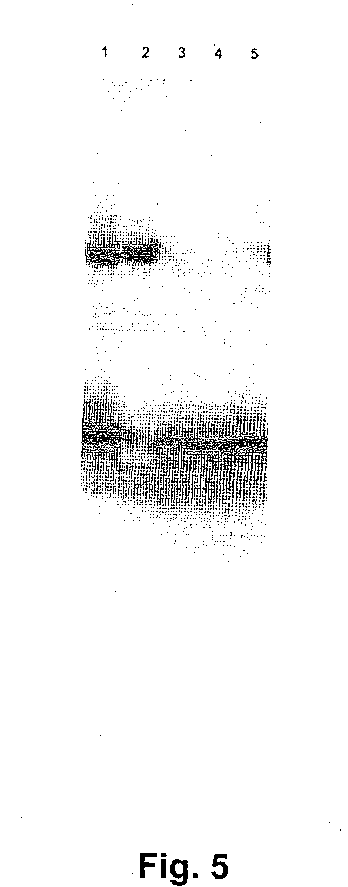

Figure 5 shows a Western blot exemplifying the expression of the fusion polypeptide Pectate lyase-ASKR-GLP1 (7-37) and the Kex2p-cleavage to free GLP1 (7-37) according to example 5. Legends to figure 5: Lane 1: Bacillus culture of Pectate lyase-ASKR-GLP1(7-37) treated with Kex protease; Lane 2: Bacillus culture of Pectate lyase-ASKR-GLP1(7-37); Lane 3: Standard of GLP1(12,5 mg/l); Lane 4: Standard of GLP1(25 mg/l); Lane 5: Standard of GLP1(50 mg/l).

### DEFINITIONS:

Prior to a discussion of the detailed embodiments of the present invention is provided a definition of specific terms related to the main aspects of the invention.

The term "a cell" or "a strain" as used herein denotes a single living cell or a population of living cells arisen from the vegetative growth of a single living cell.

The term "DNA sequence" or "DNA segment" "or DNA element" as used herein denotes the sequential order of the four bases Adenine (A), Thymine (T), Guanidine (G), and Cytidine (C) in a specific stretch of DeoxyriboNucleic Acid (DNA) which is characterized by this sequence or segment or element.

In the present context, the term "a gene" or "an open reading frame" denotes a stretch of DNA that can be expressed into a polypeptide or protein within a cell. Accordingly, said gene or open reading frame will be defined as starting from a start codon (normally "ATG", "GTG", or "TTG") and ending at a stop codon (normally "TAA", "TAG" or "TGA").

The term "a polypeptide" or "a protein" as used herein denotes the expression product resulting from the transcription by an RNA-polymerase of an open reading frame or gene into messenger-RNA which is then further translated by ribosomes via sequential addition of amino acids and linking these with peptide bonds; this process is known in the art as 'the Central Dogma'.

In order to express the gene there must be elements, as known in the art, in connection with the gene, necessary for expression of the gene within the cell. Such standard elements may include a promoter, a ribosomal binding site, a termination sequence, and may be other elements as known in the art.

In the present context, when at least two genes and maybe other DNA elements are linked together to form one single open reading frame, and these elements are expressed into one polypeptide in the same order as they are listed, the elements are said to be "sequentially fused" or "fused sequentially" and the polypeptide is referred to as a "fusion polypeptide" or "fusion protein".

The term "pectin" denotes pectate, polygalacturonic acid, and pectin which may be esterified to a higher or lower degree.

As used herein, the term "pectinase" denotes a pectinase enzyme capable of cleaving glycosidic linkages of pectic substances, mainly poly(1,4-alpha-D-galacturonide and its derivatives (see reference Sakai et al., Pectin, pectinase and protopectinase: production, properties and applications, pp 213-294 in: Advances in Applied Microbiology vol:39,1993).

The term "pectate lyase" denotes a pectinase which catalyzes the random cleavage of alpha-1,4-glycosidic linkages in pectic acid also called polygalacturonic acid by transelimination such as the enzyme class polygalacturonate lyase (EC 4.2.2.2) (PGL) also known as poly(1,4-alpha-D-galacturonide) lyase, ie also a pectate lyase which has been altered in any manner known to the skilled person, such as by amino acid deletions, insertions or substitutions, C-terminal and/or N-terminal truncations while maintaining the mentioned catalytic activity.

The term "α-amylase" or "alpha-amylase" denotes an enzyme capable of catalyzing endohydrolysis of 1,4-alpha-glucosidic linkages in oligosaccharides and polysaccharides, ie the enzymes classified as EC 3.2.1.1 according to the Enzyme Nomenclature database (http://www.expasy.ch/enzyme/).

In the present context, the term "functional hormone analogue" denotes a derivative of a naturally occurring peptide-hormone, which has retained the biological activity of the natural hormone, after this has been altered by any of the standard methods in the art such as amino acid deletions, insertions, substitutions, N- and/or C-terminal truncations. A specific example of a functional hormone analogue is Human GLP-1 (7-37) which comprises the amino acid sequence shown in positions 348 to 378 of SEQ ID 14 or in positions 356 to 386 of SEQ ID 16.

The term "Single chain Human insulin (MI3)" denotes a pro-insulin analogue as specified by the amino acid sequence shown in SEQ ID 18. This protein sequence is disclosed in the International Patent Application published as WO95/34666 which is hereby incorporated by reference in its entirety.

The term "core enzyme" denotes a single domain enzyme which may or may not have been modified or altered, but which has retained its original activity; the catalytic domain as known in the art has remained intact and functional.

As used herein, the term "target site for proteolytic cleavage" denotes an amino acid sequence which is recognized by a protease and cleaved by that protease or, alternatively, which is cleaved by treatment with a chemical compound (Current protocols in Molecular Biology. (John Wiley and Sons, 1995; Harwood, C.R., and Cutting, S. M. (eds.)).

By the term "exogenous to" or "of exogenous origin" as used herein in connection with a cell is meant a polypeptide produced by said cell due to the presence in the cell of a foreign gene, i.e. a gene which has been inserted into the cell and is not naturally occurring in this cell.

Contrary to this, by the term "native to" or "endogenous to" as used herein in connection with a specific microbial source is meant a polypeptide produced by the specific source due to the presence in the source of a native gene, i.e. a gene which has not been recombinantly inserted into a cell of the source but is naturally occurring.

By the term "linker" or "spacer" is meant a polypeptide comprising at least two amino acids which may be present between the domains of a multidomain protein, for example an enzyme comprising a core enzyme and a binding domain such as a cellulose binding domain (CBD) or any other enzyme hybrid, or between two proteins or polypeptides expressed as a fusion polypeptide, for example a fusion protein comprising two core enzymes or a fusion protein as the one present in the cell of this invention. For example, the fusion protein of two core enzymes is provided by fusing a DNA sequence encoding the first core enzyme, a DNA sequence encoding the linker and a DNA sequence encoding the second core enzyme sequentially into one open reading frame and expressing this construct. A linker may also comprise a target site for proteolytic cleavage.

### DETAILED DESCRIPTION OF THE INVENTION

### The cell

The cell of the present invention is preferably a Gram-positive cell, more preferably a *Bacillus* cell, and even more preferably selected from the group consisting of *Bacillus licheniformis, Bacillus clausii, Bacillus brevis, Bacillus amyloliquefaciens, Bacillus subtilis, Bacillus lentus, Bacillus s*t*earothermophilus, Bacillus alkalophilus, Bacillus coagulans, Bacillus circulans, Bacillus lautus, Bacillus thuringiensis,* and *Bacillus agaradhaerens*.

### The pectate lyase

Preferably, the pectate lyase present as part of the fusion protein produced by the method of the invention is selected from pectate lyases comprising an amino acid sequence selected from the group consisting of SEQ ID 2, SEQ ID 4, SEQ ID 6, SEQ ID 8, SEQ ID 10, and amino acid sequences having an amino acid sequence similarity of at least 70% any of SEQ ID 2, SEQ ID 4, SEQ ID 6, SEQ ID 8, and SEQ ID 10.

The pectate lyase shown in SEQ ID 2 (the corresponding DNA sequence is shown in SEQ ID 1) which has proven to be very beneficial for heterologous expression of polypeptides attached to the native enzyme is closely related to four other pectate lyases.

The complete DNA sequences of those four pectate lyases are presented in SEQ ID 3, SEQ ID 5, SEQ ID 7, and SEQ ID 9. These are included here as examples of other pectate sequences that could be used as fusion partners for expression of exogenous polypeptides in *Bacillus* sp. These sequences are disclosed in WO 99/27084.

The pectate lyase protein sequence could very likely be minimized to a certain extent while still retaining its beneficial characteristics for use in expression of fusion proteins. This minimization of the DNA sequence and thus the encoded protein sequence could be achieved by any known method found in the art of Molecular Biology.

One way of doing this is by specifically designing PCR primers and performing PCR to construct truncated versions of the Pectate lyase fused to the protein of interest, and test the new constructs for expression levels. Such constructs can delete part of the C-terminal end of the Pectate lyase, the N-terminal end of the pectate lyase or both ends.
Another way could be to treat the pectate lyase encoding sequence with endonucleases in such a way that the part coding for the C-terminus of the pectate lyase is degraded to various extends. A library consisting of numerous clones holding different lengths of the N-terminal part of pectate lyase fused to the peptide of interest could be screened for their expression abilities in such a way that the best expressing constructs is identified. This procedure could also be applied to the pectate lyase fusion construct in such a way that parts of the N-terminus or both the N-terminus and the C-terminus of the pectate lyase is deleted for optimal expression of the fusion protein.

### The cleaving site

The target site of proteolytic cleavage is, in a preferred embodiment of the invention, an amino acid sequence, which is recognized and cleaved by a protease.

Several amino acid sequences have been described in literature that strategically located will promote efficient cleavage of a fusion product. Most of these strategies involve site-specific proteolytic cleavage in a linker region between the mother enzyme and the wanted peptide (Polyak et al. (1997) Protein Engineering, Vol. 10 (6) pp. 615-619; Kjeldsen et al. (1996) Gene, Vol. 170 (1) pp. 107-112; Sun et al. (1995) Protein Expression and Purification, Vol. 6 (5) pp. 685-692; Martinez et al. (1995) Biochemical Journal, Vol. 306 (Pt 2) pp. 589-597.

In order to ensure efficient cleavage one could insert an amino acid sequence between the mother enzyme (in this case Pectate lyase) and the exogenous polypeptide, which codes for a recognition site for a site-specific protease. Several combinations of recognition site and proteases have been described in literature. Below we show the use of Kex2-gene encoded membrane bound proteinase from alpha-cells of *Saccharomyces cerevisiae* yeast. The Kex2 proteinase hydrolyzes peptides and proteins with basic amino acid pairs which are cleaved at the C-ends of their peptide bonds (Bessmertnaya et al. (1997) Biochemistry, Vol. 62 (8) pp. 850-857. The Kex2 cleavage site used in one preferred embodiment according to the first and second aspects is the Lys-Arg (K-/-R) sequence, but other combinations of basic amino acids could be inserted to optimize the cleavage by Kex2 (Ledgerwood. et al. (1995) J.Biochem., Vol. 308 (1) pp. 321-325; or Ghosh, S. et al. (1996) Gene (Amsterdam), Vol. 176 (1-2) pp. 249-255).

Other useful combinations of proteases and cleavage sitesare: Enterokinase (La Vallie et al. (1993) J.Biol.Chem., Vol 268 pp.2311-2317) with a preference for cleaving the amino acid sequence X-D-D-D-K-/-X, Trypsin (Jonasson et al. (1996) Eur.J.Biochem., Vol 236 (2) pp. 656-661) with a preference for cleaving the amino acid sequence X-K-R-/-X, Factor Xa (Nagai et al. (1985) PNAS, Vol 82 pp. 7252-7255) with a preference for cleaving the amino acid sequence X-I-E-G-R-/-X, Collagenase (Chinery et al. (1993) Eur.J.Biochem., Vol 212 (2) pp. 557-553) with a preference for cleaving the amino acid sequence P-X-/-G-P-X-X, Thrombin (Rahman et al. (1992) Cell.Mol.Biol., Vol 38 (5) pp. 529-542) with a preference for cleaving the amino acid sequence X-G-V-R-G-P-R-/-X, ALP (*Achromobacter lyticus* Lys-specific protease) (Kjeldsen et al., (1996) Gene, Vol 170 (1) pp. 107-112) with a preference for cleaving at Lysine, and the C-component protease from *Bacillus licheniformis* cleaving at Glu (Kakudo et al. (1992) J.Biol.Chem., Vol 267 (33) pp. 23782-23788).

Another preferred method of cleaving a peptide at a specific target site is by using chemical compounds such as cyanogen-bromide which cleaves X-M-/-X or hydroxylamine which cleaves S-N-/-G-X (Current protocols in Molecular Biology. John Wiley and Sons, 1995; Harwood, C. R., and Cutting, S. M. (eds.)).

### The linker

In a preferred embodiment of the invention, the fusion protein produced by the method of the invention comprises a linker inserted between the pectate lyase and the exogeneous polypeptide. Preferably, the linker comprises an amino acid sequence wherein at least 25% of the amino acid residues are proline. More preferred, the linker comprises at least one cycled repeat of the amino acids in sequence: Pro-Glu-Pro-Thr (PEPT, EPTP, PTEP or TPEP), and even more preferably the linker comprises at least one repeat of the amino acid sequence: Ile-Glu-Gly-Arg (IEGR).

### The exogeneous polypeptide

Usually, the exogeneous polypeptide is the desirable product of the method of the present invention. It is contemplated that the exogenous polypeptide can be any polypeptide which can be successfully expressed as part of the fusion protein produced by the method of the invention.

In a preferred embodiment of the invention, the exogeneous polypeptide is selected from the group consisting of hormones, functional hormone analogues, enzymes and artificial polypeptides.

An example of an artificial polypeptide is a single chain human insulin (MI3), preferably an insulin comprising the amino acid sequence shown in SEQ ID 18, which is encoded by a codon optimized artificial sequence shown in SEQ ID 17. The exogenous polypeptide may even be one that comprises a single chain human insulin (MI3) comprising the amino acid sequence shown in positions 356-408 in SEQ ID 20; or a MW-variant of Single chain Human insulin (MI3) comprising the amino acid sequence shown in positions 366-418 in SEQ ID 42.

It is believed that a hormone could be any peptide hormone known in the art such as the full amino acid sequence of Human GLP1.

An example of a functional hormone analogue is a Human GLP-1 (7-37) hormone analogue, preferably the analogue comprising the amino acid sequence shown in positions 348 to 378 of SEQ ID 14 or in positions 356 to 386 of SEQ ID 16.

An example of an enzyme is α-amylase. Preferred α-amylases include those disclosed in US Pat. No. 5,003,257; EP 252,666; WO/91/00353; FR 2,676,456; EP 285,123; EP 525,610; EP 368,341; and British Patent specification no. 1,296,839 (Novo).

Other amylases are stability-enhanced amylases described in WO94/18314 and WO96/05295 and amylase variants having additional modification in the immediate parent as disclosed in WO 95/10603. Also suitable are amylases described in EP 277 216, WO95/26397 and WO96/23873.

In a preferred embodiment of the invention, the α-amylase comprises the amino acid sequence shown in positions 350 to 834 of SEQ ID 12.

Examples of commercial α-amylases products are Purafect Ox Am® from Genencor and Termamyl®, Ban®, Fungamyl® and Duramyl®, all available from Novo Nordisk A/S Denmark. WO95/26397 describes other suitable amylases: α-amylases characterised by having a specific activity at least 25% higher than the specific activity of Termamyl® at a temperature range of 25°C to 55°C and at a pH value in the range of 8 to 10, measured by the Phadebas® α-amylase activity assay. Suitable are variants of the above enzymes, described in WO96/23873. Other amylolytic enzymes with improved properties with respect to the activity level and the combination of thermostability and a higher activity level are described in WO95/35382.

Preferred amylases, which can be produced by the method of the present invention, are the amylases sold under the tradename Termamyl, Duramyl and Maxamyl, the JP170 amylase comprising the amino acid sequence shown in positions 350 to 834 of SEQ ID 12, and or the a-amylase variant demonstrating increased thermostability disclosed as SEQ ID 2 in WO96/23873.

### The method for producing a protein

An essential element in this method is the use of a cell as described herein. The specific construction and cultivation of the cell permitting production of the protein may be any of the standard protocols of the art (Maniatis, T., Fritsch, E. F., Sambrook, J. "Molecular Cloning. A laboratory manual". Cold Spring Harbor Laboratories, 1982; Ausubel, F. M., et al. (eds.) "Current Protocols in Molecular Biology". John Wiley and Sons, 1995; Harwood, C. R., and Cutting, S. M. (eds.) "Molecular Biological Methods for Bacillus". John Wiley and Sons, 1990).

Similarly the specific strategy for isolating the protein of item iii) of the third aspect may be by any of the isolation protocols known to the skilled person; the isolation strategy may include cleaving the protein proteolytically in any number of ways prior to or after isolation such as known in the art.

The invention is further illustrated by the following non-limiting examples.

### MATERIALS AND METHODS

### Strains and Donor Organisms

*Bacillus licheniformis,* ATCC 14580, comprises the pectate lyase encoding DNA sequence presented in SEQ ID 1.

*E.coli* DSM 11789 comprises a plasmid containing the pectate lyase encoding DNA sequence of the invention presented in SEQ ID 1.

*E.coli* DSM 12403 comprises the plasmid containing the pectate lyase encoding DNA sequence of the invention presented in SEQ ID 3.

*E.coli* DSM 12404 comprises the plasmid containing the pectate lyase encoding DNA sequence of the invention presented in SEQ ID 5.

*E. coli* DSM 11788 comprises the plasmid containing the pectate lyase encoding DNA sequence of the invention presented in SEQ ID 7. *Bacillus sp.* KJ59, DSM 12419, comprises the pectate lyase encoding DNA sequence presented in SEQ ID 9.

*B.subtilis* PL1801. This strain is the B.subtilis DN1885 with disrupted apr and npr genes (Diderichsen, B., Wedsted, U., Hedegaard, L., Jensen, B. R., Sjøholm, C. (1990) Cloning of aldB, which encodes alpha-acetolactate decarboxylase, an exoenzyme from Bacillus brevis. J. Bacteriol., 172, 4315-4321).

*B.subtilis* WB600. This strain is a B. subtilis with six major proteases deleted. Wu, X- C., S- C. Ng, R. I. Near, and S-L. Wong. 1993. Efficient production of a functional single-chain antidigoxin antibody via an engineered Bacillus subtilis expression-secretion system. Bio/Technol. 11:71-76.

*B.subtilis* WB600asn. This strain is the WB600 strain where the amyE gene is interupted by a Tetracycline marker. Furthermore, the Chloramphenicol marker gene in WB600 is replaced with the Neomycin marker. The strain is resistant to Tetracyclin, Neomycin, Spectinomycin and Bleomycin.

*B. subtilis* PL2306. This strain is the B.subtilis DN1885 with disrupted apr and npr genes (Diderichsen, B., Wedsted, U., Hedegaard, L., Jensen, B. R., Sjøholm, C. (1990) Cloning of aldB, which encodes alpha-acetolactate decarboxylase, an exoenzyme from Bacillus brevis. J. Bacteriol., 172, 4315-4321) disrupted in the transcriptional unit of the known *Bacillus subtilis* cellulase gene, resulting in cellulase negative cells. The disruption was performed essentially as described in ( Eds. A.L. Sonenshein, J.A. Hoch and Richard Losick (1993) *Bacillus subtilis* and other Gram-Positive Bacteria, American Society for microbiology, p.618).

Competent cells were prepared and transformed as described by Yasbin, R.E., Wilson, G.A. and Young, F.E. (1975) Transformation and transfection in lysogenic strains of *Bacillus subtilis* : evidence for selective induction of prophage in competent cells. J. Bacteriol, 121:296-304.

### Plasmids

**pSJ1678** (see WO 94/19454 which is hereby incorporated by reference in its entirety).

### pMOL944:

This plasmid is a pUB110 derivative essentially containing elements making the plasmid propagatable in Bacillus subtilis, kanamycin resistance gene and having a strong promoter and signal peptide cloned from the *amyL* gene of *B.licheniformis* ATCC14580. The signal peptide contains a *Sac*II site making it convenient to clone the DNA encoding the mature part of a protein in-fusion with the signal peptide. This results in the expression of a Pre-protein which is directed towards the exterior of the cell.

The plasmid was constructed by means of conventional genetic engineering techniques which are briefly described in the following.

### Construction of pMOL944:

The pUB110 plasmid (McKenzie, T. et al., 1986, Plasmid 15:93-103) was digested with the unique restriction enzyme *Nci*I. A PCR fragment amplified from the amyL promoter encoded on the plasmid pDN1981 (P.L. Jørgensen et al., 1990, Gene, 96, p37-41.) was digested with *Nci*I and inserted in the *Nci*I digested pUB110 to give the plasmid pSJ2624.
The two PCR primers used have the following sequences:

The primer #LWN5494 (SEQ ID 21) inserts a NotI site in the plasmid.

The plasmid pSJ2624 was then digested with SacI and NotI and a new PCR fragment amplified on amyL promoter encoded on the pDN1981 was digested with *Sac*I and *Not*I and this DNA fragment was inserted in the *Sac*I-*Not*I digested pSJ2624 to give the plasmid pSJ2670.

This cloning replaces the first amyL promoter cloning with the same promoter but in the opposite direction. The two primers used for PCR amplification have the following sequences:

The plasmid pSJ2670 was digested with the restriction enzymes PstI and BclI and a PCR fragment amplified from a cloned DNA sequence encoding the alkaline amylase SP722 (disclosed in the International Patent Application published as WO95/26397 which is hereby incorporated by reference in its entirety) was digested with PstI and BclI and inserted to give the plasmid pMOL944. The two primers used for PCR amplification have the following sequence:

The primer #LWN7901 (SEQ ID 26) inserts a SacII site in the plasmid.

### PLasmid pMB541. (Construction see below)

### Propagation of donor strain.

The strain *Bacillus licheniformis* ATCC 14580 was propagated in liquid medium 3 as specified by ATCC (American Type Culture Collection, USA). After 18 hours incubation at 37°C and 300 rpm, the cells were harvested, and genomic DNA was isolated by the method described below.

### Genomic DNA Preparation

The *Bacillus licheniformis* strain was propagated in liquid media as described above. The cells were harvested, and genomic DNA was isolated by the method described by *Pitcher et al*. [*Pitcher, D. G. , Saunders, N. A., Owen, R. J;* Rapid extraction of bacterial genomic DNA with guanidium thiocyanate; Lett Appl Microbiol 1989 **8** 151-156].

### The construction pf pMB541.

### Cloning of a pectate lyase from Bacillus licheniformis.

Genomic DNA of *Bacillus licheniformis,* ATCC 14580 was partially digested with restriction enzyme *Sau*3A, and size-fractionated by electrophoresis on a 0.7 % agarose gel. Fragments between 2 and 7 kb in size were isolated by electrophoresis onto DEAE-cellulose paper (Dretzen, G., Bellard, M., Sassone-Corsi., P., Chambon, P. (1981) A reliable method for the recovery of DNA fragments from agarose and acrylamide gels. Anal. Biochem., 112, 295-298).

Isolated DNA fragments were ligated to BamHI digested pSJ1678 plasmid DNA, and the ligation mixture was used to transform *E. coli* SJ2. Transformed cells from the Genomic library of Bacillus licheniformis, ATCC 14580 were plated on LB-agar plates containing 10 µg/ml of Chloramphenicol and 0.7%, Sodium Polygalacturonate (SIGMA P-1879). The plated cells were incubated 16 hours at 37°C.

The colonies were replica plated onto fresh LB-agar plates containing 10 µg/ml of Chloramphenicol and 0.7% , Sodium Polygalacturonate (SIGMA P-1879) these plates were incubated 8 hours at 37°C. The original master plates were flooded with 5 ml 1 M CaCl2, after 5 to 30 min distinct cloudy halos appeared around putative Sodium Polygalacturonate degrading clones. The corresponding masterplate clones were picked for further characterisation. These clones were further characterized by preparing plasmid DNA from overnight 30°C liquid TY cultures of the E. coli clones and preparing plasmid DNA using Qiagen Qiaspin Prep Kit as according to manufacturer (Qiagen, Germany).

The pectate lyase positive clone *Bacillus licheniformis,* ATCC 14580 Gene library was deposited as DSM 11789. After primer walking on the plasmid of the *E.coli* DSM 11789 the SEQ ID 1 op the pectate lyase encoding DNA from *Bacillus licheniformis,* ATCC 14580 was identified.

### Identification of positive clones by activity

After incubation on plates the colonies were replica plated onto a set of LB+ 6 CAM agar plates and then further incubated at 37°C for approx. 20 hours. An overlayer containing 1% HSB agarose, 0.7% polygalacturonic acid sodium salt in an appropriate buffer was poured onto the replica plates and incubated for approx. 20 hours at 40°C. After precipitation with 5 ml 1 M CaCl2, pectate lyase positive colonies were identified after 5 to 30 min by the appearance of clear Halos at positions where pectate lyase positive clones were present.

Cells from pectate lyase positive colonies were spread for single colony isolation on agar, and a pectate lyase producing single colony was selected for each of the pectate lyase-producing colonies identified.

### Characterization of positive clones

From the restreaking plates the pectinase positive clones were obtained as single colonies, and plasmids were extracted using Qiagen Plasmid Prep as indicated by the manufacturer (Qiagen, Germany). Phenotypes were confirmed by retransformation of *E.coli* SJ2, and plasmids characterized by restriction digests.

The pectate lyase positive clone of *Bacillus licheniformis* ATCC 14580 Gene library was deposited as DSM 11789. After primer walking on the plasmid of the *E. coli* DSM 11789 the SEQ ID No 1 of the pectate lyase encoding DNA from *Bacillus licheniformis* ATCC 14580 was identified.

### Subcloning in Bacillus subtilis

The DNA encoding the mature part of the pectate lyase (represented by amino acid sequence SEQ ID 2 encoded by the DNA sequence in SEQ ID 1) of the invention was PCR amplified using the PCR primer set consisting of these two oligo nucleotides:

### Restriction sites SacII and NotI are underlined.

Chromosomal DNA isolated from *B.licheniformis* ATCC 14580 as described above was used as template in a PCR reaction using Amplitaq DNA Polymerase (Perkin Elmer) according to manufacturers instructions. The PCR reaction was set up in PCR buffer (10 mM Tris-HCl, pH 8.3, 50 mM KCl, 1.5 mM MgCl₂, 0.01 % (w/v) gelatin) containing 200 µM of each dNTP, 2.5 units of AmpliTaq polymerase (Perkin-Elmer, Cetus, USA) and 100 pmol of each primer.

The PCR reaction was performed using a DNA thermal cycler (Landgraf, Germany). One incubation at 94°C for 1 min followed by thirty cycles of PCR performed using a cycle profile of denaturation at 94°C for 30 sec, annealing at 60°C for 1 min, and extension at 72°C for 2 min. Five-µl aliquots of the amplification product was analysed by electrophoresis in 0.7 % agarose gels (NuSieve, FMC). The appearance of a DNA fragment size 1.0 kb indicated proper amplification of the gene segment.

### Subcloning of PCR fragment

The subcloning of the PCR fragment was carried out as described in example 1 except that the purified PCR fragment was digested with *Sac*II and *Not*I. One clone containing the pectate lyase gene was kept, this clone was termed MB541 and the plasmid present in MB541 is thus designated **pMB541**.

The DNA corresponding to the mature part of the pectate lyase was characterised by DNA sequencing by primerwalking, using the Taq deoxy-terminal cycle sequencing kit (Perkin-Elmer, USA), fluorescent labelled terminators and appropriate oligonucleotides as primers.

Analysis of the sequence data was performed according to Devereux et al. (1984) Nucleic Acids Res. 12, 387-395. The cloned DNA sequence was expressed in B.subtilis and the protein that appeared in the supernatant corresponded to the mature protein represented in SEQ ID 1.

### General molecular biology methods

Unless otherwise mentioned the DNA manipulations and transformations were performed using standard methods of molecular biology (Sambrook et al. (1989) Molecular cloning: A laboratory manual, Cold Spring Harbor lab., Cold Spring Harbor, NY; Ausubel, F. M. et al. (eds.) "Current protocols in Molecular Biology". John Wiley and Sons, 1995; Harwood, C. R., and Cutting, S. M. (eds.) "Molecular Biological Methods for Bacillus". John Wiley and Sons, 1990).

Enzymes for DNA manipulations were used according to the specifications of the suppliers (*e*.*g*. restriction endonucleases, ligases etc. are obtainable from New England Biolabs, Inc.).

### Media

TY (as described in Ausubel, F. M. et al. (eds.) "Current protocols in Molecular Biology". John Wiley and Sons, 1995). LB agar (as described in Ausubel, F. M. et al. (eds.) "Current protocols in Molecular Biology". John Wiley and Sons, 1995). LBPG is LB agar supplemented with 0.5% Glucose and 0.05 M potassium phosphate, pH 7.0. BPX media is described in EP 0 506 780 (WO 91/09129). CAL 18-2 media (11): Yeast extract (#0127-17-9 Difco Laboratories, MI, USA) 40g; Magnesium Sulfate (#5886 Merck, Darmstadt, Germany) 1.3g; Glucidex 12 (Roquette Feres, France) 50g; Sodium Di-hydrogenphosphate (#6346 Merck, Darmstadt, Germany) 20g; EDF-Tracemetals (recipe see below) 6.7ml; Na₂MoO₄-Tracemetals (recipe see below) 6.7ml; Pluronic PE6100 (BASF, Germany) 0.1ml; Ionexchanged water adjust to 1000ml. All is mixed, volume is adjusted, pH is measured and adjusted to pH 6.0 using NaOH. The media is sterilised by aotoclaving at 121°C for 20 min. EDF-Tracemetals (11): Mangan (II) sulphate (#5963 Merck, Darmstadt, Germany) 4.48g; Iron (III) chloride (#3943 Merck, Darmstadt, Germany) 3.33g; Copper (II) sulphate (#2790 Merck, Darmstadt, Germany) 0.625g; Zinksulphate (#8883 Merck, Darmstadt, Germany) 7.12 g; Ionexchanged water adjust to 1000ml. All is mixed, volume is adjusted. Solution is filter-sterilized and kept at 4°C. Na₂MoO₄-Tracemetals (11): SodiumMolybdat (#6521 Merck, Darmstadt, Germany) 2.0g; Ionexchanged water adjust to 1000ml. All is mixed, volume is adjusted. Solution is filtersterilized and kept at 4°C.

### Assay for alpha-amylase activity

Alpha-amylase activity was determined by a method emploing an enzymatic colorimetric test with 4,6-ethylidene(G₇)-p-nitrophenyl(G₁)-a,D-maltoheptaoside (ethylidene-G₇PNP) as substrate (Boehringer Mannheim, Germany art. 1442309). Under a specified set of conditions (temp., pH, reaction time, buffer conditions) 1 mg of a given alpha-amylase will hydrolyse a certain amount of substrate and a yellow colour will be produced. The colour intensity is measured at 405 nm. The measured absorbance is directly proportional to the activity the alpha-amylase in question under a given set of conditions.

### SDS-page and Immuno Blotting.

SDS-page was performed on a Novex (Novex, San Diego) gradient Tricine 10-20% gel under denaturing and reducing conditions as described elsewhere(SvH). Protein bands were blotted onto nitrocellulose in a Hoefer blotting module. For Immune blotting of pectatelyase-insulin fusions the monoclonal antibody F19 produced in house of Ivan Svendsen, Cell Technology was used as primary antibody and rabbit anti murine antibody coupled to peroxidase as secondary antibody (from Dako Immunoglobulins, Copenhagen, Denmark).

Immunoblotting of pectatelyase-GLP1 was done respectively with the primary antibody monoclonal aGLP 26.1 produced in house of Pia Kirschhoff Borre, Cell Technology. Binding of the peroxidase-labelled secondary antibodies was visualised by reaction with 3-amino-9-ethylcarbazol.

### EXAMPLE 1

### Construction and expression of fusion protein between Pectate lyase and JP170 alpha-amylase

The JP170 alpha-amylase encoding DNA sequence (disclosed in the International Patent Application published as WO95/26397 which is hereby incorporated by reference in its entirety) was PCR amplified using the PCR primer set consisting of the following two oligo nucleotides:

Restriction sites Sail and NheI are underlined. In the final construction the NheI site is inserted right next to the last codon in the Pectate lyase gene.

A plasmid DNA sample encoding the JP170 alpha-amylase (pMOL944) was used as template in a PCR reaction using Amplitaq DNA Polymerase (Perkin Elmer) according to manufacturers instructions. The PCR reaction was set up in PCR buffer (10 mM Tris-HCl, pH 8.3, 50 mM KCl, 1.5 mM MgCl₂, 0.01 % (w/v) gelatin) containing 200 µM of each dNTP, 2.5 units of AmpliTaq polymerase (Perkin-Elmer, Cetus, USA) and 100 pmol of each primer.

The PCR reaction was performed using a DNA thermal cycler (MJ Research, PCT-200). One incubation at 94°C for 1 min followed by thirty cycles of PCR performed using a cycle profile of denaturation at 94°C for 30 sec, annealing at 60°C for 1 min, and extension at 72°C for 2 min. Five-µl aliquots of the amplification product was analysed by electrophoresis in 0.7 % agarose gels (NuSieve, FMC). The appearance of a DNA fragment approximate size of 1.6 kb indicated proper amplification of the gene segment.

### Subcloning of PCR fragment

The purified PCR fragment coding for JP170 and the pMB541 plasmid described above was digested with SalI and NotI, and an JP170 fragment of 1639 bp and a pMB541 vector fragment of 5722 bp was purified from an agarose gel using the QIAquick Gel Extraction Kit (Qiagen, Germany). After purification, the two fragments were ligated for 16 hours at 16°C using T4 DNA ligase and buffer system (Biolab,UK). The ligation reaction was used to transform competent PL1801 and the cells were plated on LB agar plates supplemented with 10 mg Kanamycin. Several clones were analyzed by isolating plasmid DNA from overnight culture broth.

One such positive clone was restreaked several times on agar plates supplemented with 10 mg/ml Kanamycine as used above, and the clone was preserved as MOL1578. The clone MOL1578 was grown overnight in TY-10 µg/ml Kanamycin at 37°C, and next day 1 ml of cells were used to isolate plasmid from the cells using the Qiaprep Spin Plasmid Miniprep Kit #27106 according to the manufacturers recommendations for B.subtilis plasmid preparations. The purified plasmid was DNA sequenced and revealed the DNA sequence corresponding to the fusionprotein of: Pectate lyase-JP170. The plasmid was named pMOL1578. The total DNA sequence of the ORF encoding the Pectate lyase-JP170 is represented in SEQ ID 11 and the derived protein sequence is found in SEQ ID 12. In SEQ ID 12 the amino acids have the following origin and features:

| | | |
|---|---|---|
| a.a. | 1-29 | The signalpeptide of amyL from *B. licheniformis.* |
| a.a. | 30-343 | The pectate lyase from *B.licheniformis*. |
| a.a. | 344-345 | Two ammino acids derived from the cloning site NheI. |
| a.a. | 346-349 | IEGR linker. |
| a.a. | 350-834 | The JP170 amylase. |

### Expression and detection of Pectate lyase-JP170 fusion protein

MOL1578 coding for the Pectate-lyase-JP170 hybrid and the two reference strains MOL944 coding for JP170 and MB541 coding for Pectate lyase was incubated for 5 days in BPX-medium at 30°C and 300 rpm. 100 ml of cell-free supernatant was mixed 100 ml of SDS loading buffer and 25 µl was loaded on a 4-20% Laemmli Tris-Glycine, SDS-PAGE NO:VEX gel (Novex, USA). The samples were electrophoresed in a Xcell™ Mini-Cell (NO:VEX, USA) as recommended by the manufacturer, all subsequent handling of gels including staining with comassie, destaining and drying were performed as described by the manufacturer.

The appearance of a protein band of approx. 90 kDa, indicated expression in *B. subtilis* of the Pectate lyase-JP170 fusion encoded on the plasmid pMOL1578. However, the major bands on the gel corresponds to each of the two core enzymes Pectate lyase (35 kDa) and JP170 amylase (55 kDa) indicating that the fusion protein is being processed during or immediately after translocation.

Samples from each of the three strains MOL1578, MOL944 and MB541 were analysed for alpha-amylase activity according to the procedure described above to determine the yields of JP170. Table 1 show a significant increase of amylase yields of 2.2 fold in the MOL1578 strain with the Pectate lyase-JP170 fusion when compared to the MOL944 strain coding for JP170 alone. In conclusion it is evident that the Pectate lyase can be used as a secretion enhancer for large enzymes such as the JP170 amylase.

**Table 1**

| **Strain** | **Enzyme** | **Units** |
|---|---|---|
| MOL944a | JP170 | 0.93 |
| MOL944b | JP170 | 1.26 |
| MOL1578a | Pectate lyase-JP170 | 2.32 |
| MOL1578b | Pectate lyase-JP170 | 2.52 |
| MB541a | Pectate lyase | 0.1 |
| MB541b | Pectate lyase | 0.1 |

### EXAMPLE 2

### Construction and expression of fusion protein between Pectate lyase and GLP-1

The Human GLP-1 hormone normalizes blood glucose level in type 2 diabetics (WO9517510-A1). The GLP-1(7-37) analogue is 31 amino acids long and is difficult to produce by traditional techniques in yeast (Egel-Mitani et al. (in press) Yield improvement of various heterologous peptides expressed in YPS1-disrupted *Saccharomyces cerevisiae* strains).

The GLP-1 gene was amplified using the overlapping primer set below. A DNA sequence coding for a specific Kex2 recognition site Lys-Arg (KR) was inserted just upstream from the first codon of GLP-1(7-37) to allow for specific cleavage between the Pectate lyase and GLP-1(7-37). (see reference to Kex2 in for example: Ledgerwood. et al. (1995) Biochemical Journal, Vol. 308 (1) pp. 321-325, or Ghosh, S. et al. (1996)Gene (Amsterdam), Vol. 176 (1-2) pp. 249-255. ). The following two primers were used to amplify the GLP-1(7-37)-Kex2 sequence: The overlap extention reaction was set up in PCR buffer (10 mM Tris-HCl, pH 8.3, 50 mM KCl, 1.5 mM MgCl₂, 0.01 % (w/v) gelatin) containing 200 µM of each dNTP, 2.5 units of AmpliTaq polymerase (Perkin-Elmer, Cetus, USA) and 100 pMol of each primer

The overlap extention reaction was performed using a DNA thermal cycler (MJ Research, PCT-200). One incubation at 94°C for 1 min followed by fifteen cycles performed using a cycle profile of, annealing at 55°C for 1 min, and extension at 72°C for 0.5 min. Five-µl aliquots of the amplification product was analysed by electrophoresis in 2.0 % agarose gels (NuSieve, FMC). The appearance of a DNA fragment of the size of 129 bp indicated proper amplification.

### Subcloning of the GLP1 fragment

The pMOL1578 plasmid was used as vektor for cloning the GLP-1(7-37) sequence in frame to make a fusion construct. The GLP-1(7-37) fragment and the pMOL1578 vector was digested with NheI and EagI. The GLP-1(7-37) fragent of 119 bp and the vector fragment of 5779 bp were purified and cloned as described for the pMOL1578 construction.

One positive PL1801 transformed clone was restreaked several times on agar plates supplemented with 10 mg/ml Kanamycine as used above, and. the clone was preserved as MOL1635. The clone MOL1635 was grown overnight in TY-10 µg/ml Kanamycin at 37°C, and next day 1 ml of cells were used to isolate plasmid from the cells using the Qiaprep Spin Plasmid Miniprep Kit #27106 according to the manufacturers recommendations for B.subtilis plasmid preparations. The purified plasmid was DNA sequenced and revealed the DNA sequence corresponding to the fusion protein of: Pectate lyase-GLP-1(7-37). The plasmid was named pMOL1621.

The total DNA sequence of the ORF encoding the Pectate lyase- GLP-1(7-37) is represented in SEQ ID 13 and the derived protein sequence is found in SEQ ID 14. In SEQ ID 14 the aminoacids have the following origin and features:

| | | |
|---|---|---|
| a.a. | 1-29 | The signalpeptide of amyL from *B.licheniformis.* |
| a.a. | 30-343 | The pectate lyase from *B.licheniformis.* |
| a.a. | 344-345 | Two amino acids derived from the cloning site *Nhe*I. |
| a.a. | 346-347 | The Kex 2 endoprotease processing site. |
| a.a. | 348-378 | The Human GLP-1(7-37) sequence |

### Expression and detection of Pectate lyase- GLP-1(7-37) fusion protein

The plasmid pMOL1621 was used to transform the protease weak *Bacillus subtilis* strain WB600asn. One such strain of WB600asn with pMOL1621 was termed MOL1636. This strain was cultivated in media Cal18-2 with 10 µg/ml of Kanamycin, 100 ml of media in a 500 ml shake flask with two baffles were inoculated with approximately 10E8 cells and incubated for 24 hours at 37°C at 300 rpm. Samples were taken, culture broth was spun and cell-free supernatants were recovered and analysed by conventional Western blotting (See figures 1 and 2).

### EXAMPLE 3

### Construction and expression of fusion protein between Pectate lyase and GLP1 including a PEPT linker

In order to avoid proteolytic attack in the region between the Pectate lyase and GLP-1(7-37) a stable linker region was inserted. The linker sequence of choice was the PEPT motif which has been reported in patent WO 99/01543. The linker motif was present in the patented cellulase between the mother enzyme and the CBD (Celulase Binding Domain) and in the current invention the PEPT linker has prooven to be stable towards proteolysis in *Bacillus subtilis.*

To ensure a proper spacing between the Pectate lyase and GLP-1 the motif was repeated twice as PEPTPEPT (2x(PEPT)). A DNA sequence coding for a specific Kex2 recognition site Lys-Arg (KR) was inserted just upstream from the first codon of GLP-1 (7-37) to allow for specific cleavage between the Pectate lyase and GLP-1 (7-37) after purification (see reference to Kex2 in for example: Ledgerwood. et al. (1995) Biochemical Journal, Vol. 308 (1) pp. 321-325, or Ghosh, S. et al. (1996) Gene (Amsterdam), Vol. 176 (1-2) pp. 249-255.).

Two separate PCR reactions were performed using pMOL1621 as template. PCR conditions were as described in example 1. The sequence of the two primer pairs were as follows:

### 1. PRIMER PAIR

### 2.PRIMER PAIR

The two PCR reactions were purified using Qiagen columns (QIAquick PCR Purification Kit #28106) as 220 bp and 400 bp fragments. An SOE (Sequence Overlap Extention) reaction) was set up in PCR conditions as described above using 10 ng of each fragment. The PCR cycle was performed as, one incubation at 94°C for 1 min followed by ten cycles using a cycle profile of, denaturation at 94°C for 10 sec., annealing at 55°C for 30 sec., and extension at 72°C for 2 min. 100 pmol of the flanking primers 142670.forward (SEQ ID 36) and 101450.reverse (SEQ ID 34) was added to the reaction and the PCR cycle was continued for another 20 cycles. Five-µl aliquots of the amplification product was analysed by electrophoresis in 2.0 % agarose gels (NuSieve, FMC). The appearance of a DNA fragment of the size of 600 bp indicated proper amplification.

### Subcloning of the GLP1 fragment

The pMOL1578 plasmid was used as vektor for cloning the 2x(PEPT)- GLP-1 (7-37) gene described above in frame to make a fusion construct. The 600 bp PCR fragment holding the PEPT-GLP-1 (7-37) sequence and the pMOL1578 vector was digested with NheI and EagI. The PEPT- GLP-1 (7-37) fragment of 137 bp and the vector fragment of 5779 bp were purified and cloned as decribed for the pMOL1578 construction.

One positive WB600asn transformed clone was restreaked several times on agar plates supplemented with 10 mg/ml Kanamycine as used above, and the clone was preserved as MOL1698. The clone MOL1698 was grown overnight in TY-10 µg/ml Kanamycin at 37°C, and next day 1 ml of cells were used to isolate plasmid from the cells using the Qiaprep Spin Plasmid Miniprep Kit #27106 according to the manufacturers recommendations for B.subtilis plasmid preparations. The purified plasmid was DNA sequenced and revealed the DNA sequence corresponding to the fusion protein of: Pectate lyase-linker-GLP1. The plasmid was named pMOL1698.

The total DNA sequence of the ORF encoding the Pectate lyase-linker- is represented in SEQ ID 15 and the derived protein sequence is found in SEQ ID 16. In SEQ ID 16 the aminoacids have the following origin and features:

| | | |
|---|---|---|
| a.a. | 1-29 | The signalpeptide of amyL from *B.licheniformis.* |
| a.a. | 30-343 | The pectate lyase from *B.licheniformis.* |
| a.a. | 344-345 | Two ammino acids derived from the cloning site NheI. |
| a.a. | 346-353 | The PEPTPEPT linker region |
| a.a. | 354-355 | The Kex 2 endoprotease processing site. |
| a.a. | 356-386 | The Human GLP-1 (7-37) sequence |

Expression and detection of Pectate lyase-linker-GLP-1 (7-37) fusion protein

MOL1698 coding for the Pectate-lyase hybrid was cultivated in media Cal18-2 with 10 µg/ml of Kanamycin. In 500 ml shake flasks with two baffles 100 ml of media was inoculated with approximately 10E8 cells and incubated for 24 hours at 37°C at 300 rpm. Samples were taken, culture broths were spun and cell-free supernatants were recovered and analysed by conventional Western blotting and 25 µl was loaded on a 4-20% Laemmli Tris-Glycine, SDS-PAGE NO: VEX gel (Novex, USA). The samples were electrophoresed in a Xcell™ Mini-Cell (NO:VEX, USA) as recommended by the manufacturer, all subsequent handling of gels including staining with comassie, destaining and drying were performed as described by the manufacturer (see: Figure 3).

### EXAMPLE 4

### Construction and expression of fusion protein between Pectate lyase and Single chain Human insulin (MI3)

The DNA sequence encoding the human insulin MI3 was derived from reverse transcribing the protein sequence of this as specified by the sequence SEQ ID 18. A reference to one patent application covering this Protein sequence is made to WO95/34666. The DNA sequence was further optimized for codon usage approaching the codon usage found in the Pectate lyase described in SEQ ID 1. This was done simply by using the most preferred codons of the pectate lyase SEQ ID 1 as the codons for encoding the MI3 molecule found in SEQ ID 18. The preferred DNA sequence for this experiment can be found in SEQ ID 17.

In order to have a flexible region (linker) between the Pectate lyase and The MI3 we used a linker composed of PEPT repeats. Specifically for this experiment we used a two repeat of this PEPT. Again the codons were optimized to approach the codon usage of the Pectatelyase.

In order to have a possibility to cleave the MI3 from the pectate lyase after having purified the fusion protein from the supernatant of cultured cells, a Kex 2 endoprotease processing site was introduced just after the linker and prior to the MI3 protein. (see reference to Kex2 in for example Ledgerwood. et al. (1995) Biochemical Journal, Vol. 308 (1) pp. 321-325, or Ghosh, S. et al. (1996)Gene (Amsterdam), Vol. 176 (1-2) pp. 249-255. ) The Kex2 site used here is Lys-Arg (KR). The construction of the Pectate lyase-Linker-Kex-MI3 encoding plasmid was done as follows:

Two overlapping oligonucleotides were designed in such a way that they, when used on each other in a PCR reaction would result in the formation of a DNA fragment primarily consisting of the sequence found in SEQ ID 17, a linker encoding sequence, a protease cleavage site encoding sequence and two restriction endonuclease sites necesarry for subcloning of the DNA fragment.

The construction of the DNA fragment was as follows: The two overlapping oligonucleotides:

### Restriction sites NheI and NotI are underlined.

The oligonucleotides were used in a PCR reaction in HiFidelityTM PCR buffer (Boehringer Mannheim, Germany) supplemented with 200 µM of each dNTP, 2.6 units of HiFidelityTM Expand enzyme mix and 200 pmol of each primer

The PCR reaction was performed using a DNA thermal cycler (Landgraf, Germany). One incubation at 94°C for 1 min followed by ten cycles of PCR performed using a cycle profile of denaturation at 94°C for 15 sec, annealing at 60°C for 60 sec, and extension at 72°C for 120sec, followed by twenty cycles of denaturation at 94°C for 15 sec, 60°C for 60 sec and 72°C for 120 sec (at this elongation step 20 sec are added every cycle). Five µl aliquots of the amplification product were analyzed by electrophoresis in 1.5 % agarose gels (NuSieve, FMC) with ReadyLoad 100bp DNA ladder (GibcoBRL, Denmark) as a size marker. A clear DNA fragment of 0.2 kb indicated proper assembly of the two primers.

Fortyfive µl aliquots of the PCR products generated as described above were purified using QIAquick PCR purification kit (Qiagen, USA) according to the manufacturer's instructions. The purified DNA was eluted in 50 µl of 10mM Tris-HCl, pH 8.5.

Five µg of pMOL1578 and twentyfive µl of the purified PCR fragment was digested with NheI and NotI, the digested pMOL1578 was electrophoresed in 0.8 % low gelling temperature agarose (SeaPlaque GTG, FMC) gels, the relevant fragment was excised from the gels, and purified using QIAquick Gel extraction Kit (Qiagen, USA) according to the manufacturer's instructions. The isolated PCR DNA fragment was after digestion simply purified using QIAquick PCR purification kit (Qiagen, USA) according to the manufacturer's instructions. The purified DNA was eluted in 50 µl of 10mM Tris-HCl, pH 8.5.
The PCR fragment and plasmid were then ligated to the NheI-NotI digested and purified pMOL1578. The ligation was performed overnight at 16°C using 0.5 µg of each DNA fragment, 1 U of T4 DNA ligase and T4 ligase buffer (Boehringer Mannheim, Germany).

The ligation mixture was used to transform competent *B.subtilis* PL2306. The transformed cells were plated onto LBPG-10 µg/ml of Kanamycin plates. After 18 hours incubation at 37°C several clones were restreaked on fresh agar plates and also grown in liquid TY cultures with 10 µg/ ml kanamycin and incubated overnight at 37°C. Next day 1 ml of cells were used to isolate plasmid from the cells using the Qiaprep Spin Plasmid Miniprep Kit #27106 according to the manufacturers recommendations for *B.subtilis* plasmid preparations. This plasmid DNA was used as template for DNA sequencing.

Two clones containing the DNA sequence corresponding to the pectate lyase in fusion with the linker and MI3 gene was kept, these clones were termed MB929-1 and MB929-3. The DNA sequence of the ORF encoding the Signalpeptide-Pectatelyase-linker-MI3 is represented in SEQ ID 19 and the derived protein sequence is found in SEQ ID 20.

### In SEQ ID 20 the following amino acids have the following features:

| | | |
|---|---|---|
| a.a. | 1-29 | The signalpeptide of amyL from *B.licheniformis*. |
| a.a. | 30-343 | The pectate lyase from *B.licheniformis*. |
| a.a. | 344-345 | Two ammino acids derived from the cloning site *Nhe*I. |
| a.a. | 346-353 | The PEPT linker. |
| a.a. | 354-355 | The Kex 2 endoprotease processing site. |
| a.a. | 356-408 | The Human single chain insulin MI3. |

From the same cloning as above a clone was isolated which did not make full length Pectatelyase-linker-MI3, this clone was termed pMB929-5. By DNA sequencing it was shown that there was a stop codon introduced just after the PEPT-KRFVN sequence. This clone was used as control in expression and detection analysis.

### Expression of Pectate lyase-Linker-MI3 fusion protein.

The pMB929 plasmids were used to transform the protease weak Bacillus subtilis strain WB600asn. One such strain of WB600asn with pMB929-1 was termed MB1009-1. One such strain of WB600asn with pMB929-3 was termed MB1009-4. One such strain of WB600asn with pMB929-5 was termed MB1009-7 and was used as the negative control strain (as described above). These strains were cultivated in media Cal18-2 with 10 µg/ml of Kanamycin. In 500 ml shake flasks with two baffles 100 ml of media was inoculated with approximately 10E8 cells and incubated for 24 hours at 37°C at 300 rpm. Samples were taken, culture broths were spun and cell-free supernatants were recovered and analysed by conventional Western blotting (see: Figure 4).

### Example 5

### Expression and Kex2p cleavage of fusion protein between Pectate lyase and GLP-1

The protease weak strain WB600asn was transformed with the plasmid pMOL1621 to prepare for overexpression and cleavage of the Pectate lyase-ASKR-GLPl (7-37) fusion product. This strain was cultivated in media Cal18-2 with 10 µg/ml of Kanamycin, 100 ml of media in a 500 ml shake flask with two baffles were inoculated with approximately 10E8 cells and incubated for 24 hours at 37°C at 300 rpm. Samples were taken, culture broth were spun and cell-free supernatants were recovered and analysed. The Kex2p-cleavage was performed as follows:
a) 0.8 ml of supernatant
b) 0.1 ml of 1 M Na₂HPO₄, pH 7.5
c) 0.1 ml of Kex2p is added (Preparation no KMK0087, a gift from Steen B. Mortensen, Novo Nordisk)
d) Incubation for 2 hours in a waterbath at 37°C
e) Samples quickly frozen and stored at -20°C.

The samples were thawed immidiately before the SDS-PAGE run. 30 µl sample was treated with 20 µl sample buffer and 5 µl PMSF (dissolved in isopropanol) and 5 µl 25 % w/v DTT before the boiling . Ten µl was applied in the wells. The individual samples are described in the worksheet. All other conditions were as described before in materials and methods.
Figure 5 demonstrates that the Pectate lyase-ASKR-GLPl (7-37) fusion protein was efficiently cleaved by Kex2p to leave a GLP1 product recognized by the GLP1 antibody. Yields of GLP1 was of the order of 50 mg/l.

## Claims

1. A cell comprising a DNA sequence coding for at least the following elements fused sequentially into one open reading frame, a pectate lyase, a target site for proteolytic cleavage, and a polypeptide of exogenous origin.

2. A cell comprising a DNA sequence coding for at least the following elements fused sequentially into one open reading frame, a pectate lyase, a linker of at least 2 amino acids, a target site for proteolytic cleavage, and a polypeptide of exogenous origin.

3. The cell according to claim 1 or 2, wherein the cell is a Gram-positive microbial cell.

4. The cell according to any of claims 1 to 3, wherein the cell is a Bacillus cell.

5. The cell according to any of claims 1 to 4, wherein the cell is selected from the group consisting of *Bacillus licheniformis, Bacillus clausii, Bacillus brevis, Bacillus amyloliquefaciens, Bacillus subtilis, Bacillus lentus, Bacillus stearothermophilus, Bacillus alkalophilus, Bacillus coagulans, Bacillus circulans, Bacillus lautus, Bacillus thuringiensis,* and *Bacillus agaradhaerens.*

6. The cell according to any of claims 1 to 5, wherein the target site for proteolytic cleavage is an amino acid sequence that is recognized and cleaved by a protease.

7. The cell according to claim 6, wherein the target site for proteolytic cleavage is the amino acid sequence Lys-Arg (KR).

8. The cell according to claim 6, wherein the target site for proteolytic cleavage is the amino acid sequence Ile-Glu-Gly-Arg (IEGR).

9. The cell according to any of claims 1 to 5, wherein the target site for proteolytic cleavage is an amino acid sequence that is cleaved when the fused polypeptide is secreted by the cell.

10. The cell according to any of claims 1 to 5, wherein the target site for proteolytic cleavage is an amino acid sequence capable of being cleaved by a chemical compound.

11. The cell according to claim 10, wherein the chemical compound is cyanogen-bromide or hydroxylamine.

12. The cell according to any of claims 1 to 11, wherein the pectate lyase is selected from the group of pectate lyases comprising an amino acid sequence selected from the group consisting of SEQ ID 2, SEQ ID 4, SEQ ID 6, SEQ ID 8, and SEQ ID 10.

13. The cell according to any of claims 1 to 12, wherein the pectate lyase is a homologue with an amino acid sequence similarity of 70% with a pectate lyase comprising an amino acid sequence selected from the group consisting of SEQ ID 2, SEQ ID 4, SEQ ID 6, SEQ ID 8, and SEQ ID 10.

14. The cell according to any of claims 2 to 13, wherein at least 25% of the amino acid residues present in the linker are proline.

15. The cell according to any of claims 2 to 14, wherein the linker comprises at least one cycled repeat of the amino acids in sequence: Pro-Glu-Pro-Thr, Glu-Pro-Thr-Pro, Pro-Thr-Pro-Glu, or Thr-Pro-Glu-Pro (PEPT, EPTP, PTEP or TPEP).

16. The cell according to any of claims 2 to 13, wherein the linker comprises at least one repeat of the amino acid sequence Ile-Glu-Gly-Arg (IEGR).

17. The cell according to any of claims 1 to 16, wherein the exogenous polypeptide is selected from the group consisting of hormones, functional hormone analogues, enzymes and artificial polypeptides.

18. The cell according to any of claims 1 to 16, wherein the exogenous polypeptide is an α-amylase comprising the amino acid sequence shown in positions 350 to 834 of SEQ ID 12.

19. The cell according to any of claims 1 to 16, wherein the exogenous polypeptide is a Human GLP-1 (7-37) hormone analogue comprising the amino acid sequence shown in positions 348 to 378 of SEQ ID 14 or in positions 356 to 386 of SEQ ID 16.

20. The cell according to any of claims 1 to 16, wherein the exogenous polypeptide is a single chain human insulin (MI3) comprising the amino acid sequence shown in SEQ ID 18.

21. The cell according to any of claims 1 to 16, wherein the exogenous polypeptide comprises a single chain human insulin (MI3) comprising the amino acid sequence shown in positions 356-408 in SEQ ID 20.

22. A method for producing a protein, the method comprising the steps of:
i) constructing a suitable cell comprising a DNA sequence coding for at least the following sequential elements fused into one open reading frame (ORF): a pectate lyase, target site for proteolytic cleavage, and a polypeptide of exogenous origin,
ii) culturing the cell constructed in step (i) under suitable conditions for growth and secretion, and
iii) recovering the protein that comprises a polypeptide of exogenous origin.

23. The method according to claim 22, which further comprises the step of proteolytically cleaving the protein prior to or after recovering the polypeptide of exogenous origin.

## Patentansprüche

1. Zelle, umfassend eine DNA-Sequenz, die für wenigstens die folgenden, der Reihe nach in einem offenen Leserahmen fusionierten Elemente kodiert, eine Pektat-Lyase, ein Zielort für eine proteolytische Spaltung und ein Polypeptid exogenen Ursprungs.

2. Zelle, umfassend eine DNA-Sequenz, die für wenigstens die folgenden, der Reihe nach in einem offenen Leserahmen fusionierten Elemente kodiert, eine Pektat-Lyase, ein Linker von wenigstens 2 Aminosäuren, ein Zielort für eine proteolytische Spaltung und ein Polypeptid exogenen Ursprungs.

3. Zelle nach Anspruch 1 oder 2, wobei die Zelle eine Gram-positive mikrobielle Zelle ist.

4. Zelle nach einem beliebigen der Ansprüche 1 bis 3, wobei die Zelle eine *Bacillus*-Zelle ist.

5. Zelle nach einem beliebigen der Ansprüche 1 bis 4, wobei die Zelle aus der Gruppe, bestehend aus *Bacillus licheniformis, Bacillus clausii, Bacillus brevis, Bacillus amyloliquefaciens, Bacillus subtilis, Bacillus lentus, Bacillus stearothermophilus, Bacillus alkalophilus, Bacillus coagulans, Bacillus circulans, Bacillus lautus, Bacillus thuringiensis* und *Bacillus agaradhaerens,* ausgewählt ist.

6. Zelle nach einem beliebigen der Ansprüche 1 bis 5, wobei der Zielort für eine proteolytische Spaltung eine Aminosäuresequenz ist, die von einer Protease erkannt und gespalten wird.

7. Zelle nach Anspruch 6, wobei der Zielort für eine proteolytische Spaltung die Aminosäuresequenz Lys-Arg (KR) ist.

8. Zelle nach Anspruch 6, wobei der Zielort für eine proteolytische Spaltung die Aminosäuresequenz Ile-Glu-Gly-Arg (IEGR) ist.

9. Zelle nach einem beliebigen der Ansprüche 1 bis 5, wobei der Zielort für eine proteolytische Spaltung eine Aminosäuresequenz ist, die gespalten wird, wenn das fusionierte Polypeptid von der Zelle sekretiert wird.

10. Zelle nach einem beliebigen der Ansprüche 1 bis 5, wobei der Zielort für eine proteolytische Spaltung eine Aminosäuresequenz ist, die in der Lage ist, von einer chemischen Verbindung gespalten zu werden.

11. Zelle nach Anspruch 10, wobei die chemische Verbindung Bromcyan oder Hydroxylamin ist.

12. Zelle nach einem beliebigen der Ansprüche 1 bis 11, wobei die Pectat-Lyase aus der Gruppe von Pektat-Lyasen ausgewählt ist, die eine Aminosäuresequenz umfassen, die aus der Gruppe, bestehend aus SEQ ID 2, SEQ ID 4, SEQ ID 6, SEQ ID 8 und SEQ ID 10, ausgewählt ist.

13. Zelle nach einem beliebigen der Ansprüche 1 bis 12, wobei die Pectat-Lyase ein Homolog mit einer Aminosäuresequenz-Ähnlichkeit von 70 % zu einer Pektat-Lyase ist, die eine aus der Gruppe, bestehend aus SEQ ID 2, SEQ ID 4, SEQ ID 6, SEQ ID 8 und SEQ ID 10, ausgewählte Aminosäuresequenz umfasst.

14. Zelle nach einem beliebigen der Ansprüche 2 bis 13, wobei wenigstens 25 % der in dem Linker vorhandenen Aminosäurereste Prolin sind.

15. Zelle nach einem beliebigen der Ansprüche 2 bis 14, wobei der Linker wenigstens eine zyklisierte Wiederholung der Aminosäuren in der Sequenz: Pro-Glu-Pro-Thr, Glu-Pro-Thr-Pro, Pro-Thr-Pro-Glu oder Thr-Pro-Glu-Pro (PEPT, EPTP, PTEP oder TPEP) umfasst.

16. Zelle nach einem beliebigen der Ansprüche 2 bis 13, wobei der Linker wenigstens eine Wiederholung der Aminosäuresequenz lle-Glu-Gly-Arg (IEGR) umfasst.

17. Zelle nach einem beliebigen der Ansprüche 1 bis 16, wobei das exogene Polypeptid aus der Gruppe, bestehend aus Hormonen, funktionellen Hormonanaloga, Enzymen und künstlichen Polypeptiden, ausgewählt ist.

18. Zelle nach einem beliebigen der Ansprüche 1 bis 16, wobei das exogene Polypeptid eine die an den Positionen 350 bis 834 von SEQ ID 12 gezeigte Aminosäuresequenz umfassende α-Amylase ist.

19. Zelle nach einem beliebigen der Ansprüche 1 bis 16, wobei das exogene Polypeptid ein Humanes-GLP-1-(7-37)-Hormonanalog ist, das die an den Positionen 348 bis 378 von SEQ ID 14 oder an den Positionen 356 bis 386 von SEQ ID 16 gezeigte Aminosäuresequenz umfasst.

20. Zelle nach einem beliebigen der Ansprüche 1 bis 16, wobei das exogene Polypeptid ein die in SEQ ID 18 gezeigte Aminosäuresequenz umfassendes, einzelkettiges humanes Insulin (MI3) ist.

21. Zelle nach einem beliebigen der Ansprüche 1 bis 16, wobei das exogene Polypeptid ein die an den Positionen 356-408 in SEQ ID 20 gezeigte Aminosäuresequenz umfassendes, einzelkettiges humanes Insulin (MI3) umfasst.

22. Verfahren zur Herstellung eines Proteins, wobei das Verfahren die Schritte umfasst:
i) Konstruieren einer geeigneten Zelle, die eine DNA-Sequenz umfasst, die für wenigstens die folgenden aufeinanderfolgenden, in einem offenen Leserahmen (ORF) fusionierten Elemente kodiert: eine-Pectat-Lyase, ein Zielort für eine proteolytische Spaltung und ein Polypeptid exogenen Ursprungs,
ii) Kultivieren der in Schritt (i) konstruierten Zelle unter geeigneten Bedingungen für das Wachstum und die Sekretion, und
iii) Gewinnen des Proteins, das ein Polypeptid exogenen Ursprungs umfasst.

23. Verfahren nach Anspruch 22, das außerdem den Schritt des proteolytischen Spaltens des Proteins vor oder nach dem Gewinnen des Polypeptids exogenen Ursprungs umfasst.

## Revendications

1. Cellule comprenant une séquence d'ADN codant pour au moins les éléments suivants fusionnés séquentiellement en un cadre de lecture ouvert, une pectate lyase, un site cible pour un clivage protéolytique et un polypeptide d'origine exogène.

2. Cellule comprenant une séquence d'ADN codant pour au moins les éléments suivants fusionnés séquentiellement en un cadre de lecture ouvert, une pectate lyase, un lieur d'au moins 2 acides aminés, un site cible pour un clivage protéolytique et un polypeptide d'origine exogène.

3. Cellule selon a revendication 1 ou 2, dans laquelle la cellule est une cellule microbienne Gram positive.

4. Cellule selon l'une quelconque des revendications 1 à 3, dans laquelle la cellule est une cellule *Bacillus.*

5. Cellule selon l'une quelconque des revendications 1 à 4, dans laquelle la cellule est choisie dans le groupe comprenant *Bacillus licheniformis, Bacillus clausii, Bacillus brevis, Bacillus amyloliquefaciens, Bacillus subtilis, Bacillus lentus, Bacillus stearothermophilus, Bacillus alkalophilus, Bacillus coagulans, Bacillus circulans, Bacillus lautus, Bacillus thuringiensis et Bacillus agaradhaerens.*

6. Cellule selon l'une quelconque des revendications 1 à 5, dans laquelle le site cible pour un clivage protéolytique est une séquence d'acides aminés qui est reconnue et clivée par une protéase.

7. Cellule selon la revendication 6, dans laquelle le site cible pour un clivage protéolytique est la séquence d'acides aminés Lys-Arg (KR).

8. Cellule selon la revendication 6, dans laquelle le site cible pour un clivage protéolytique est la séquence d'acides aminés Ile-Glu-Gly-Arg (IEGR).

9. Cellule selon l'une quelconque des revendications 1 à 5, dans laquelle le site cible pour un clivage protéolytique est une séquence d'acides aminés qui est clivée lorsque le polypeptide fusionné est sécrété par la cellule.

10. Cellule selon l'une quelconque des revendications 1 à 5, dans laquelle le site cible pour un clivage protéolytique est une séquence d'acides aminés capable d'être clivée par un composé chimique.

11. Cellule selon la revendication 10, dans laquelle le composé chimique est un cyanogène-bromure ou une hydroxylamine.

12. Cellule selon l'une quelconque des revendications 1 à 11, dans laquelle la pectate lyase est choisie dans le groupe de pectate lyases comprenant une séquence d'acides aminés choisie dans le groupe comprenant SEQ ID 2, SEQ ID 4, SEQ ID 6, SEQ ID 8 et SEQ ID 10.

13. Cellule selon l'une quelconque des revendications 1 à 12, dans laquelle la pectate lyase est un homologue ayant une similarité de séquence d'acides aminés de 70 % avec une pectate lyase comprenant une séquence d'acides aminés choisie dans le groupe comprenant SEQ ID 2, SEQ ID 4, SEQ ID 6, SEQ ID 8 et SEQ ID 10.

14. Cellule selon l'une quelconque des revendications 2 à 13, dans laquelle au moins 25 % des résidus d'acides aminés présents dans le lieur sont la proline.

15. Cellule selon l'une quelconque des revendications 2 à 14, dans laquelle le lieur comprend au moins une répétition cyclique des acides aminés en séquence : Pro-Glu-Pro-Thr, Glu-Pro-Thr-Pro, Pro-Thr-Pro-Glu ou Thr-Pro-Glu-Pro (PEPT, EPTP, PTEP ou TPEP).

16. Cellule selon l'une quelconque des revendications 2 à 13, dans laquelle le lieur comprend au moins une répétition de la séquence d'acides aminés Ile-Glu-Gly-Arg (IEGR).

17. Cellule selon l'une quelconque des revendications 1 à 16, dans laquelle le polypeptide exogène est choisi dans le groupe comprenant des hormones, des analogues d'hormones fonctionnelles, des enzymes et des polypeptides artificiels.

18. Cellule selon l'une quelconque des revendications 1 à 16, dans laquelle le polypeptide exogène est une α-amylase comprenant la séquence d'acides aminés représentée aux positions 350 à 834 de SEQ ID 12.

19. Cellule selon l'une quelconque des revendications 1 à 16, dans laquelle le polypeptide exogène est un analogue d'hormone GLP-1 (7-37) humaine comprenant la séquence d'acides aminés représentée aux positions 348 à 378 de SEQ ID 14 ou aux positions 356 à 386 de SEQ ID 16.

20. Cellule selon l'une quelconque des revendications 1 à 16, dans laquelle le polypeptide exogène est une insuline humaine (MI3) à chaîne unique comprenant la séquence d'acides aminés représentée dans SEQ ID 18.

21. Cellule selon l'une quelconque des revendications 1 à 16, dans laquelle le polypeptide exogène comprend une insuline humaine (MI3) à chaîne unique comprenant la séquence d'acides aminés représentée aux positions 356 à 408 dans SEQ ID 20.

22. Procédé destiné à la production d'une protéine, le procédé comprenant les étapes de :
i) construire une cellule adaptée comprenant une séquence d'ADN codant pour au moins les éléments séquentiels suivants fusionnés en un cadre de lecture ouvert (ORF) : une pectate lyase, un site cible pour un clivage protéolyique et un polypeptide d'origine exogène,
ii) cultiver la cellule construite dans l'étape (i) dans des conditions adaptées pour la croissance et la sécrétion, et
iii) récupérer la protéine comprenant un polypeptide d'origine exogène.

23. Procédé selon la revendication 22, qui comprend en outre l'étape de clivage protéolytique de la protéine avant ou après la récupération du polypeptide d'origine exogène.
